# NEUE EUROPÄISCHE PATENTSCHRIFT

(11) **EP 2 459 163 B2**
(45) Veröffentlichungstag und Bekanntmachung des Hinweises auf die Entscheidung über den Einspruch: **16.04.2025**
(45) Hinweis auf die Patenterteilung: 04.04.2018
(21) Anmeldenummer: 10726519.1
(22) Anmeldetag: 30.06.2010
(51) Int. Cl.: A61K 8/73, A61Q 17/00, A61Q 19/10, A61Q 19/00

(54) **HAUTSCHUTZMITTEL, INSEBSONDERE GEGEN HYDROPHOBE (LIPOPHILE) UND HYDROPHILE (LIPOPHOBE) SCHADSTOFFE**
SKIN CARE COMPOSITIONS, IN PARTICULAR AGAINST HYDROPHOBIC (LIPOPHILIC) AND HYDROPHILIC (LYPOPHOBIC) CONTAMINANTS
COMPOSITIONS DE PROTECTION DE LA PEAU, PARTICULIEREMENT CONTRE DES SUBSTANCES NOCIVES HYDROPHOBES (LIPOPHILES) ET HYDROPHILES (LIPOPHOBES)

(30) Priorität: 31.07.2009 DE 102009028143
(43) Veröffentlichungstag der Anmeldung: 06.06.2012
(73) Patentinhaber: Deb IP Limited, Denby, Derbyshire DE5 8JZ (GB)
(72) Erfinder: THÖRNER, Brigitte, 40235 Düsseldorf (DE); ALLEF, Petra, 45147 Essen (DE); SCHMIDT, Christian, 21037 Hamburg (DE); VEEGER, Marcel, 47574 Goch (DE); SCHMIDT, Stefani, 21037 Hamburg (DE); KLÖREN, Tanja, 47839 Krefeld (DE); CLAASSEN, Anne, 47906 Kempen (DE)
(74) Vertreter: Thurston, Joanna
(86) Internationale Anmeldenummer: PCT/EP2010/059244
(87) Internationale Veröffentlichungsnummer: WO 2011/012394

(56) Entgegenhaltungen:
- EP-B1- 1 066 366
- WO-A1-2005/016364
- WO-A2-01/70271
- US-A- 4 900 550
- STOCKHAUSEN: "Travabon und Travabon S", 1 January 2005 (2005-01-01), pages 1 - 6, XP002593712, Retrieved from the Internet <URL:http://www.leber-gmbh.de/Downloads/Datenblaetter/Hautschutz/Travabon.pdf> [retrieved on 20100727]
- STOCKHAUSEN: "Travabon und Travabon S", January 2005 (2005-01-01), XP002593712, Retrieved from the Internet <URL:http://www.leber-gmbh.de/Downloads/Datenblaetter/Hautschutz/Travabon.pdf> [retrieved on 20100727]
- GREVEN HAUTSCHUTZ: "Ligana Oleo-tec", 10 June 2008 (2008-06-10), XP002593713, Retrieved from the Internet <URL:http://www.peter-greven-hautschutz.de/fileadmin/user_upload/produkte/D_LIGANA_OLEO_TEC_CREME_A_V3.pdf> [retrieved on 20100727]
- GREVEN HAUTSCHUTZ: "Ligana Mult-tec", 10 June 2008 (2008-06-10), XP002593714, Retrieved from the Internet <URL:http://www.peter-greven-hautschutz.de/fileadmin/user_upload/produkte/D_LIGANA_MULTI_TEC_LOTION_WA_V2.pdf> [retrieved on 20100727]
- J. KRESKEN: "Hautschutz am Arbeitsplatz", October 1999 (1999-10-01), XP002593715, Retrieved from the Internet <URL:http://www.scf-online.com/german/ausgabe21/workplace_21_d.htm> [retrieved on 20100727]

## Beschreibung

Die Erfindung betrifft ein Hautschutzmittel, insbesondere gegen ölige (hydrophobe) Schadstoffe, das auch bei Wechselbelastung, d.h. bei Belastung von sowohl hydrophilen als auch hydrophoben Schadstoffen und bei starker Schmutzbelastung einsetzbar ist.

Die menschliche Haut ist mit einer Oberfläche von ca. 1,5 bis 2,0 m² das oberflächengrößte Organ des Menschen, das für den Körper lebenswichtige Funktionen wahrnimmt. Hierfür enthält die Haut Blut- und Lymphgefäße, durch deren Wände hindurch der Austausch von Lymphflüssigkeit, Gasen, Nähr- und Abfallstoffen stattfinden kann, um z.B. Ernährung und Stoffwechsel zu gewährleisten. Weitere Funktionen der Haut sind die Regelung der Körpertemperatur, der Schutz des Körpers gegen Austrocknung und gegen äußere mechanische, chemische und bakterielle Einwirkungen. So halten die Sekrete von Hauttalgdrüsen die Haut geschmeidig und helfen bei der Regulierung des Wasserhaushaltes der Haut. Darüber hinaus vermittelt die Haut unter anderem über freie Nervenendigungen dem Organismus Tast-, Wärme- und Kälte- und Schmerzreize und hat somit die Funktion eines Sinnesorgans.

Die mannigfaltigen für Hygiene, Körperpflege und Kosmetik angebotenen Produkte, wie z.B. Hautreinigungs-, Hautschutz- und Hautpflegeprodukte dienen daher nicht nur primär der Reinigung, dem Schutz bzw. der Pflege der Haut, sondern sie erhalten auch die Lebensqualität und sichern das Wohlbefinden des Menschen.

Als Körperhülle stellt die menschliche Haut die Verbindung, aber zugleich auch Abgrenzung des menschlichen Körpers mit seiner Außenwelt dar. Insbesondere erfolgt über sie die Kontaktaufnahme mit allem, was der menschliche Organismus zum Leben benötigt - aber auch was sie gefährden kann.

Insbesondere am Arbeitsplatz kann die Haut einer Vielzahl von Arbeitsstoffen unterschiedlichster Art ausgesetzt sein und auch Arbeitsstoffe, die gemeinhin nicht als Gefahrstoffe gelten, können bei Langzeitkontakt zu Hautschäden wie z.B. Hauttrockenheit, spröder rissiger Haut mit gestörter Barrierewirkung und / oder erhöhtem Allergierisiko führen. Neben solchen Langzeiteinflüssen bereits schwach irritierender Substanzen sowie der Vielfältigkeit der am Arbeitsplatz eingesetzten Arbeitsstoffe müssen Hautschutzmittel dem Umstand Rechnung tragen, dass die Haut des Menschen individuell verschieden auf äußere Umwelteinflüsse reagiert bzw. hierdurch dann individuell starke Unterschiede der Hautreaktion auf diese Einflüsse beobachtet werden.

Hautschutzmittel können entsprechend ihren Einsatzgebieten in folgende Kategorien eingeteilt werden (vgl. Dr. Jens Burfeindt, Dr. Dirk Mehlan, Dr. Wolfgang Röcher in "sicher ist sicher" - Arbeitsschutz aktuell, 12 (2006), 547 - 551):
Schutz vor Arbeitsstoffen
Erleichterung der Hautreinigung
Schutz vor Quellung und Hornhauterweichung bei Feuchtarbeit
Schutz vor UV-Strahlen

Weitere Anforderungen, die an moderne Hautschutzmittel gestellt werden, sind insbesondere, dass die Griffigkeit der Hände möglichst wenig beeinträchtigt wird bzw. es muss gewährleistet sein, dass die verwendeten Hautschutzmittel mit den jeweiligen Arbeitsmaterialien bzw. - prozessen, wie sie beispielsweise in der Automobil- und Lackindustrie sowie der gummiverarbeitenden Industrie vorkommen, verträglich sind, d.h. sie müssen z.B. silikonfrei, fettfrei, fettarm etc. sein:
So ist beispielsweise besonders nachteilig an Hautschutzpräparaten, die Silikone als Inhaltsstoffe enthalten, dass diese Präparate auf Gegenständen z.B. auf Werkstoffen bzw. Werkstücken Rückstände hinterlassen können, wenn solche Werkstücke per Hand einem weiteren Arbeitsgang zugeführt werden. Daher können bei zu lackierenden Werkstücken die Beschäftigten solche silikonhaltigen Hautschutzmittel nicht einsetzen. Diese sehr schwer entfernbaren Silikonrückstände wirken nämlich bei der Weiterverarbeitung dieser Werkstücke wie z. B. Lackieren oder Vulkanisieren stark störend und führen zu Lackierungsfehlern wie z.B. Lackbenetzungsstörungen bzw. Kraterbildung auf den Werkstücken. Somit können, insbesondere in der Automobil- und Lackindustrie sowie der gummiverarbeitenden Industrie silikonhaltige Hautschutzprodukte trotz ihrer hervorragenden Schutzwirkung und Akzeptanz beim Anwender nicht zum Einsatz kommen.

Darüber hinaus müssen Hautschutzmittel frei von bekannten penetrationsfördernden Inhaltsstoffen wie z.B. Harnstoff sein.

Weiterhin sollen aus ökologischen Gründen moderne Hautschutzmittel auch frei von fluorhaltigen Tensiden sein. Solche Fluortenside enthaltenden Hautschutzmittel sind nicht nur ökologisch bedenklich, sondern können ebenfalls - wie im Falle der oben beschriebenen silikonhaltigen Hautschutzmittel - bei der Weiterverarbeitung von Werkstücken zu Lackierungsfehlern führen, wenn sich entsprechende Hautschutzpräparatrückstände auf den zu lackierenden Werkstücken befunden haben.

Die Wirkung eines Hautschutzmittels beruht erfahrungsgemäß auf einem Zusammenspiel physikalischer und physiologischer Effekte der einzelnen Inhaltsstoffe in der Gesamtformulierung. Insbesondere macht man sich die seit dem Altertum bekannte allgemeine Regel "Similia similibus solvuntur" ("Ähnliches wird von Ähnlichem gelöst") zunutze und setzt z.B. bei hydrophilen (lipophoben) Stoffen ein Hautschutzmittel ein, das ein entgegengesetzes Eigenschaftsprofil aufweist, also hydrophob (lipophil) ist. Darüberhinaus können Pigmente oder filmbildende Substanzen die Diffusion eines Arbeitsstoffes in die Haut verzögern und somit die Gefahr eines schädigenden Effektes für die Haut vermindern.

Als besonders vorteilhafte Hautschutzmittel sind auch solche Mittel zu nennen, die neben ihrer Schutzwirkung gegen Schadstoffe auch die Haftung stark haftender Verschmutzungen wie z.B. wasserunlöslicher Verschmutzungen durch Öle, Lacke, Graphit, Metallstäube, Klebestoffe, Kunstharze etc. vermindern, oder durch spezielle Inhaltsstoffe selbst eine Reinigungswirkung ausüben. Hierdurch kann die anschließende Hautreinigung wesentlich schneller und hautschonender durchgeführt werden, was sich natürlich positiv auf den Hautzustand auswirkt. Solche besonders vorteilhaften Hautschutzmittel werden von der Fa. Evonik Stockhausen GmbH, Krefeld unter der Markenbezeichnung TRAVABON^{®} im Markt vertrieben. Entsprechend sind bereits Produktbeschreibungen und entsprechende Zusammensetzungen dem Fachmann bekannt, und als Internet-Publikationen auffindbar (URL:http://www.leber-gmbh.de/downloads/datenblaetter/hautschutz/travabon.pdf).

Hautschutzmittel sollen die menschliche Haut vor den unterschiedlichsten Gefährdungen der Außenwelt schützen, wie z.B. Witterungseinflüsse, Wasser und wässrige Lösungen, Chemikalien sowie Verschmutzungen jedweder Art. Üblicherweise überziehen solche Hautschutzmittel in wasserfreier Form die menschliche Haut mit einem "Barriere"- bzw. Schutzfilm, der von der Haut nicht absorbiert werden kann. Idealerweise ist dieser Schutzfilm dergestalt auf der Haut appliziert, dass sein Vorhandensein von der Umwelt nicht bemerkt wird, da er für diese unsichtbar bzw. für den Anwender auf dessen Haut nicht spürbar ist. Dennoch enthalten die meisten am Markt erhältlichen Produkte große Mengen - beispielsweise bis zu 25 Gew.-% - an saugfähigen, gut deckenden sowie an der Haut gut haftenden, ungiftigen anorganischen Feststoffen in Pigmentform. Exemplarisch seien hier Oxide, wie z.B. Zink- und Titandioxid, die Erdalkalicarbonate und -hydrogencarbonate sowie - sulfate, insbesondere deren Magnesium-, Calcium- und Strontiumsalze genannt. Weiterhin sind neben Siliziumdioxid sowie Kieselgelen mineralische Silikate wie z.B. Aluminiumsilikate, Magnesium-Aluminium-Silicate, Bentonite, Kaoline und Talkum zu nennen. Solche anorganischen Pigmente wirken als Barrieremittel, in dem sie auf der Haut eine physikalische Schutzschicht ausbilden. Solche anorganischen Barrieremittel wie z.B. Kaolin oder Talkum vermitteln jedoch dem Anwender ein sehr unangenehmes trockenes Hautgefühl. Bei großer Hitze bzw. wenn der Anwender schwitzt oder er Handschuhe trägt, fühlt sich die Haut "klebrig" an. Hierdurch wird die Akzeptanz der Anwender hinsichtlich des Einsatzes solcher Hautschutzmittel am Arbeitsplatz deutlich herabgesetzt.

Darüber hinaus werden im Handel Hautschutzmittel angeboten, die sowohl gegen hydrophile (lipophobe) als auch gegen hydrophobe (lipophile) Schadstoffe Schutz bieten sollen, um einen gewissen Schutz für die Haut auch bei häufig wechselnder Beanspruchung ermöglichen zu können. Nichtsdestoweniger sind solche ambivalenten Hautschutzmittel oftmals in ihrer Wirksamkeit eingeschränkt im Vergleich zu Hautschutzmitteln, deren Einsatzspektrum sich lediglich entweder ausschließlich auf hydrophile (lipophobe) oder ausschließlich auf hydrophobe (lipophile) Schadstoffe beschränkt.

Weitere im Handel erhältliche Hautschutzmittel sind Mittel mit adstringierenden Eigenschaften, die durch die Verwendung von speziellen Gerbstoffen als Inhaltsstoffen deren adstringierende Wirkung dergestalt nutzen, dass die obersten Zellen der Hornhaut verfestigt werden. Hierdurch wird eine Erhöhung der mechanischen Belastbarkeit und der Barrierewirkung der Haut erreicht. Hieraus resultiert die Eignung solcher Hautschutzmittel sowohl bei erhöhter Beanspruchung der Haut durch mechanische Einwirkungen als auch bei häufig wechselnder Hautbelastung durch wässrige und ölige Stoffe.

In Stand der Technik sind entsprechend einige Hautschutzmittel, welche pflegende oder Hautabdichtene bzw. barrierebildende Substanzen enthalten bekannt. Diesbezüglich offenbart WO2001/70271 ein Hautschutzmittel enthaltend neben Gycerin und Wasser unter anderem Carrageneenan, Johannisbrotmehl und Algin. Weiterhin offenbart WO 2005016364 ein Hautschutzmittel, das neben Glycerin und Wasser Chondrus Crispus enthält und in US 4,900,550 wird ein Hautschutzmittel beschrieben, das neben Wasser und Polyglycol und Polysorbat auch Carrageen, Gummi Arabicum, Tragacanth und Johannisbrotmehl. Eine weitere Schutzcreme enthaltend Barriere-bildende Substanzen und gleichzeitig wirksam gegen öllösliche Arbeitsstoffe ist bekannt unter dem Handelsnamen "Ligana Mulit Tec", und wird von der Firma Greven Hautschutz GmbH & Co. KG vertrieben. Entsprechende Produktinformationen sind auf der Internetseite (www.peter-greven.com) zu finden.

Wie bereits zuvor gesagt kann die Haut am Arbeitsplatz einer Vielzahl von Arbeitsstoffen unterschiedlichster Art ausgesetzt sein, wobei auch Arbeitsstoffe, die gemeinhin nicht als Gefahrstoffe gelten, bei Langzeiteinfluss zu Hautschädigungen führen können. Es hat sich jedoch gezeigt, dass im Hinblick auf die gesamte Bandbreite an denkbaren Arbeitsstoffen und den daraus resultierenden vielfältigen Einsatzgebieten von Hautschutzmitteln am Arbeitsplatz gerade ein immer größer werdender Bedarf an einem Hautschutzmittel besteht, das insbesondere eine verbesserte Schutzwirkung für die Haut auch bei häufig wechselnder Beanspruchung, d.h. Schutz sowohl gegen hydrophile (lipophobe) als auch gegen hydrophobe (lipophile) Schadstoffe bietet und eine vergleichbare Reinigungswirkung bzw. einen vergleichbaren Schutz vor Hautverschmutzungen bei der Anwendung zeigt.

Nichtsdestoweniger sollen diese Hautschutzmittel dem Anwender ein verbessertes - sprich "ein angenehmeres" Hautgefühl vermitteln als die im Handel erhältlichen Hautschutzprodukte mit physikalischen Barrieremitteln, wie z.B. Kaolin, Talkum etc.

Um einen Schutz vor Schmutz zu bieten, sprich eine vereinfachte Reinigung zu gewährleisten, werden dann den Produkten Tenside zugesetzt. Die meisten Tenside erhöhen allerdings die Penetration von Schadstoffen, so dass bis jetzt kein Hautschutzmittel auf dem Markt erhältlich ist, das einen Schutz vor öligen und wässrigen Noxen und einen Schutz vor Schmutz bietet.

Aufgabe war es daher, ein Hautschutzmittel, welches auch bei häufig wechselnder Schadstoffbeanspruchung, insbesondere durch hydrophobe (lipophile) als auch hydrophile (lipophobe) Schadstoffe, eine vorzugsweise mindestens vergleichbarer Reinigungswirkung wie die im Stand der Technik bekannten Hautschutzmittel aufweist sowie bei der Anwendung vorzugsweise ein "angenehmes" Hautgefühl bewirkt, und welche vorzugsweise fluortensid- und silikonfrei ist, sowie ein entsprechendes Herstellungsverfahren eines solchen Hautschutzmittels bereitzustellen.

Die erfindungsgemäße Aufgabe wurde durch ein Hautschutzmittel, insbesondere gegen hydrophobe (lipophile) als auch gegen hydrophile (lipophobe) Schadstoffe gemäß Anspruch 1 gelöst. Bevorzugte Ausführungsformen der Erfindung sind Gegenstand der Unteransprüche.

Es war völlig überraschend, dass hydrophobe Polymere, d.h. Polymere, die sich nur trüb in Wasser lösen, mit einem nephelometrischen Trübungswert größer 40, als barrierebildende Komponente in Hautschutzmitteln einen guten Schutz gegen ölige Noxen bieten. Darüber hinaus schützt das erfindungsgemäße Hautschutzmittel nicht nur gegen ölige, hydrophobe (liphophile) Schadstoffe, sondern auch bei Wechselbelastung, d.h. bei Belastung von sowohl hydro- philen als auch hydrophoben Schadstoffen und bei starker Schmutzbelastung. Demgegenüber war zu erwarten, dass hydrophobe Polymere als barrierebildende Komponenten in Hautschutzmitteln ausschließlich vor hydrophilen (lipophoben) Schadstoffen, d.h. vor wäßrigen Noxen schützen. Dass diese in den erfindungsgemäßen Hautschutzmitteln ebenfalls auch vor hydrophoben (lipophilen) Schadstoffen, insbesondere öligen Noxen schützen, war völlig unerwartet.

Gegenstand der vorliegenden Erfindung sind deshalb Hautschutzmittel wie in den Ansprüchen und nachfolgend beschrieben, deren Verwendung als Schutzmittel gegen hydrophile (lipophobe) Schadstoffe bzw. als Schutzmittel sowohl gegen hydrophobe (lipophile) als auch gegen hydrophile (lipophobe) Schadstoffe und ggf. zur Erleichterung der Hautreinigung nach Verschmutzung sowie ein Verfahren zur Herstellung entsprechender Hautschutzmittel.

Die erfindungsgemäßen Hautschutzmittel haben den Vorteil, dass sie auch ohne das Vorhandensein von Silikon- oder Fluortensiden einen guten Schutz vor lipophilen Noxen bieten. Durch den Verzicht der genannten Tenside werden die bekannten Nachteile des versehentlichen Übertragens der Lipohilie bzw. Hydrophobie auf angefasste Werkstücke vermieden.

Die erfindungsgemäßen Hautschutzmittel haben außerdem den Vorteil, dass ihre Anwendung nicht nur einen guten Schutz vor lipophilen Noxen sondern auch vor hydrophilen Noxen bieten kann. Die erfindungsgemäßen Hautschutzmittel bieten deshalb einen besonders guten Schutz bei Tätigkeiten, bei denen sowohl ein Kontakt mit lipohilen als auch mit hydrophilen Noxen Vorkommen kann.

Die erfindungsgemäßen Hautschutzmittel und deren Verwendung sowie ein Verfahren zu deren Herstellung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Wenn nicht anders angegeben sind bei Angaben in Prozent immer Massen-% bzw. Gew.-% gemeint. Sind nachfolgend Mittelwerte angegeben, so sind diese, wenn nicht anders angegeben, die zahlengemittelten Mittelwerte. Wird im Rahmen der vorliegenden Erfindung von Lösungen von Polymeren in Wasser gesprochen, so sind darunter auch solche Lösungen, Dispersionen und/oder Suspensionen zu verstehen, in denen Teile der Polymeren gelöst und Teile der Polymeren ungelöst oder gequollen vorliegen.

Das erfindungsgemäße Hautschutzmittel, insbesondere gegen hydrophobe (lipophile) Schadstoffe, zeichnet sich dadurch aus, dass es mindestens eine barrierebildende Komponente, wobei eine 1 gew.-%ige Lösung dieser barrierebildenden Komponente in Wasser einen nephelometrischen Trübungswert, bestimmt mittels Turbidimetrie, größer 40 (NTU), vorzugsweise größer 50 und bevorzugt größer 60 aufweist, enthält.

Der nephemologische Trübungswert kann z. B. unter Verwendung eines Turbidimeters vom Typ HACH Turbidimeter 2100P ISO der Fa. Hach Company, Loveland, Colorado (USA) unter Verwendung von Rundküvetten mit Schraubverschluss der Fa. Hach Company, Loveland, Colorado (USA), Katalog-Nummer 24347-06 und unter Verwendung der Kalibriersusbstanzen StablCal Solution HACH (< 0,1 NTU), Katalognummer 26597-42, StablCal Solution HACH (20 NTU), Katalognummer 26601-42, StablCal Solution HACH (100 NTU), Katalognummer 26602-42 und StablCal Solution HACH (800 NTU), Katalognummer 26605-42, alle ebenfalls von der Firma Hach Company, bestimmt werden.

Der Anteil an barrierebildenden Komponenten, deren 1 gew.-%ige Lösung in Wasser einen nephelometrischen Trübungswert wie oben angegeben aufweist, an der Gesamtzusammensetzung des erfindungsgemäßen Hautschutzmittels (NTU) beträgt vorzugsweise von 0,01 bis 5 Gew.-%, bevorzugt von 0,03 bis 5 Gew.-% und besonders bevorzugt von 0,03 bis 3 Gew.-%.

In dem erfindungsgemäßen Hautschutzmittel kann jede in kosmetischen Rezepturen einsetzbare barrierebildende Komponente, deren 1 gew.-%ige Lösung in Wasser einen nephelometrischen Trübungswert in den oben angegebenen Bereichen aufweist, eingesetzt werden.

Bevorzugt sind in dem erfindungsgemäßen Hautschutzmittel ein oder mehrere natürlich vorkommende und/oder synthetisch hergestellte, voprzugsweise natürlich vorkommende Polymere, deren 1 gew.-%ige Lösung in Wasser einen nephelometrischen Trübungswert in den oben angegebenen Bereichen aufweisen, als barrierebildende Komponente enthalten.

Als barrierebildende Komponenten einsetzbare Polymere, deren 1 gew.-%ige Lösung in Wasser einen nephelometrischen Trübungswert in den oben angegebenen Bereichen aufweisen, sind besonders bevorzugt Polysaccharide in den erfindungsgemäßen Hautschutzmitteln enthalten, die besonders vorteilhaft aus der Gruppe der Gummen ausgewählt sein können. Zu den Gummen zählt man z. B. Pflanzen- oder Baumsäfte, die an der Luft erhärten und Harze bilden oder Extrakte aus Wasserpflanzen. Aus dieser Gruppe können erfindungsgemäß insbesondere Gummi Arabicum, Johannisbrotmehl, Tragacanth, Karaya, Guar Gummi, Pektin, Gellan Gummi, Carrageen, Agar, Algine, Chondrus, Xanthan Gummi etc. oder Mischungen daraus ausgewählt werden.

Weitere besonders bevorzugte Polysaccharide sind insbesondere Alginate, d.h. Salze und Ester der Alginsäure, vorzugsweise Natriumalginat bzw. Chondrus Crispus (Carrageen) sowie das unter der INCI-Bezeichnung Biosaccharide Gum-4 bekannte Pflanzengummi.

Als barrierebildende Komponenten, deren 1 gew.-%ige Lösung in Wasser einen nephelometrischen Trübungswert in den oben angegebenen Bereichen aufweisen, einsetzbare Polymere sind des Weiteren bevorzugt Polyurethane und Polyacrylate.

Um zu gewährleisten, dass die erfindungsgemäßen Hautschutzmittel einen Schutz vor Hautverschmutzungen bieten und auch eine vergleichbare Reinigungswirkung zeigen wie die im Stand der Technik bekannten Hautschutzprodukte, kann es vorteilhaft sein, wenn die erfindungsgemäßen Hautschutzmittel mindestens ein Tensid enthalten.

Prinzipiell können in dem erfindungsgemäßen Hautschutzmittel alle Tenside, insbesondere für die Anwendung in kosmetischen Mitteln geeigneten Tenside eingesetzt werden. Hierbei sollte berücksichtigt werden, dass Tenside für die menschliche Haut per se Noxen darstellen können bzw. manche Tenside überdies penetrationsfördernd sein können, so dass dies dem für ein Hautschutzmittel gewünschten Effekt, nämlich gerade die Penetration von Schadstoffen in die Haut zu verhindern, zuwider läuft.

Somit werden in den erfindungsgemäßen Hautschutzmitteln vorzugsweise nur solche Tenside eingesetzt, bei denen sichergestellt ist, dass hierdurch die Schutzwirkung des Mittels als ganzes nicht beeinträchtigt ist. Weiterhin sollten die einsetzbaren Tenside eine gute Hautverträglichkeit aufweisen, insbesondere auch deshalb, weil es sich bei Hautschutzmitteln um "Leave on" Produkte handelt bzw. das Mittel über einen längeren Zeitraum auf der Haut des Anwenders verbleiben soll.

Besonders bevorzugte Tenside sind deshalb milde bzw. hautverträgliche Tenside, die allein oder in Kombination in den erfindungsgemäßen Hautschutzmitteln enthalten sein können.

Solche Tenside sind beispielsweise Aminosäuretenside, insbesondere Acylaminosäuren und deren Salze, wie
- Acylglutamate, beispielsweise Natriumacylglutamat, Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Soja Protein und Natrium-/Kalium Cocoyl hydrolysiertes Kollagen, Sarcosinate, beispielsweise Myristoyl Sarcosin, TEA-lauroyl Sarcosinat, Natriumlauroylsarcosinat und Natriumcocoylsarkosinat,
   -Acyltauride, beispielsweise Natriumlauroyltaurat und Natriummethylcocoyltaurat, -Isethionate, insbesondere Acylisethionate mit 8 bis 24 C-Atomen, z. B. Natrium-/Ammoniumcocoyl-isethionat,
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat.

Weitere einsetzbare milde Tenside können zwitterionische Tenside, insbesondere die sogenannten Betaine, wie z.B. die N-Alkyl-N,N-dimethylammonium-glycinate, beispielsweise das **Kokosalkyl**-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein besonders bevorzugtes zwitterionisches Tensid ist das unter der INCI-Bezeichnung Cocamidopropyl Betaine bekannte Fettsäureamid-Derivat.

Erfindungsgemäß bevorzugt einsetzbare Tenside sind amphotere Tenside, insbesondere -Acyl-/dialkylethylendiamin, beispielsweise Natriumacylamphoacetat, Dinatriumacylamphodipropionat,Dinatriumalkylamphodiacetat, Natriumacylamphohydroxypropylsulfonat, Dinatriumacylamphodiacetat und Natriumacylamphopropionat,
-N-Alkylaminosäuren, beispielsweise Aminopropylalkylglutamid, Alkylaminopropionsäure, Natriumalkylimidodipropionat und Lauroamphocarboxyglycinat.
-N-Alkyl- oder N-Alkenylbetaine mit mindestens 8 C-Atomen, wie z. B. Laurylamidopropylbetain und Oleylamidopropylbetain.

Erfindungsgemäß besonders bevorzugt einsetzbare Tenside sind Polyethylen- und Polypropylenglycolderivate, insbesondere Polypropylenglycolether, die vorzugsweise mindestens 6 C-Atome aufweisen, wie beispielsweise Laureth-6 oder Oleth-5.

Ganz besonders bevorzugte Tenside, von denen vorzugsweise mindestens eines in den erfindungsgemäßen Hautschutzmitteln enthalten ist, sind vorzugsweise ausgewählt aus der Gruppe umfassend: Natrium Stearoyl Glutamat, Natrium Cocoamphoacetat, Dinatrium Cocoyl Glutamat, Natrium Cocoamphopropionat, Dioctyl Natrium Sulfosuccinat, und Natrium Lauryl Sarkosinat.

Die erfindungsgemäßen Hautschutzmittel enthalten vorzugsweise von 1 bis 12 Gew.-%, bevorzugt 1 bis 10 Gew.-% und ganz besonders bevorzugt 1 bis 8 Gew.-% Aktivsubstanz, bezogen auf die Gesamtzusammensetzung des Hautschutzmittels, mindestens eines, vorzugsweise milden Tensids, bevorzugt ausgewählt aus den vorstehend genannten Tensiden.

In den erfindungsgemäßen Hautschutzmitteln können Polyole sowie deren Derivate, insbesondere von 0,1 bis 5 Gew.-%, bevorzugt von 1 bis 5 Gew.-% und ganz besonders bevorzugt von 2 bis 4 Gew.-%, bezogen auf die Gesamtzusammensetzung des Hautschutzmittels, z. B. als Moisturizer, enthalten sein. Überraschend war, dass Propylenglykol, das als penetrationsfödernd bekannt ist, keine negativen Eigenschaften auf die Schutzwirkung zeigt.

Als Polyole, die allein oder als Mischung mit einem weiteren oder mehreren anderen Polyolen eingesetzt werden können, werden erfindungsgemäß alle Verbindungen verstanden, die unter die in Römpp's Lexikon der Chemie, Version 3.4 Römpp Online, Georg Thieme Verlag angegebenen Definition fallen. Insbesondere kommen übliche für kosmetische und/oder pharmazeutische Formulierungen geeignete, d.h. physiologisch verträgliche Polyalkohole bzw. Polyhydoxyverbindungen, vorzugsweise mit 2 bis 15 Kohlenstoffatomen und mindestens 2 Hydroxylgruppen in betracht. Exemplarisch sind hier mehrwertige Alkohole wie geradkettige, verzweigte oder cyclische Alkanole mit 2 bis 15, vorzugsweise 2 bis 6 Kohlenstoffatomen zu nennen, wobei Glyzerin und/oder 1,2 Propandiol besonders bevorzugt sind. Darüber hinaus können Glykole, wie z.B. Polyethylenglykole, Polypropylenglykole aber auch Zucker und Zuckerderivate, vorzugsweise Fructose, Glucose, Saccharose, Zuckeralkohole, insbesondere Sorbit, Mannit etc. in den erfindungsgemäßen Hautschutzmitteln enthalten sein. Die Polyole bzw. Polyolderivate weisen vorzugsweise freie OH-Gruppen auf und sind damit bevorzugt keine Verbindungen, deren OH-Gruppen verethert sind.

Als Komponente weist das erfindungsgemäße Hautschutzmittel vorzugsweise außerdem Wasser auf. Vorzugsweise beträgt der Anteil an Wasser an der Gesamtzusammensetzung von 50 bis 90 Gew.-%, bezogen auf die Gesamtzusammensetzung des Hautschutzmittels.

Als weitere Komponenten können die erfindungsgemäßen Hautschutzmittel gegebenenfalls, Hilfs-, Zusatz- und/oder Wirkstoffe wie z.B. Farbstoffe, Lösungsvermittler, Komplexbildner, Gerbstoffe, Pigmente, Sequestrierungsmittel, Lichtschutzfilter oder Parfüm- bzw. Duftstoffe, pH-Regulatoren, Stabilisatoren, Konservierungsmittel, beispielsweise Parabene, d.h. p-Hydroxybenzoesäurealkylester, wie z.B. Methyl-, Ethyl-, Propyl- und/oder Butylparaben, sowie Phenoxyethanol etc., Antioxidantien und/oder ölige oder wässrige Pflegekomponenten als weitere Komponenten enthalten. Vorzugsweise liegt der Anteil dieser weiteren Komponenten in den für kosmetische und/oder pharmazeutische Formulierungen üblichen Mengen. Bevorzugt beträgt der Anteil der weiteren Komponenten von 0,01 bis 25 Gew.-%, vorzugsweise 0,05 bis 10 Gew.-%, bezogen auf das Gesamtgewicht des Hautschutzmittels, wobei der Fachmann den Gewichtsanteil dieser Komponenten so wählt, dass es zu keiner Beeinträchtigung der Barrierebildung der erfindungsgemäßen Hautschutzmittel aufgrund möglicher penetrationsfördernden Eigenschaften dieser Hilfs-, Zusatz- und/oder Wirkstoffe kommt.

Obgleich die erfindungsgemäßen Hautschutzmittel eine hervorragende Schutzwirkung bei Belastung von sowohl hydrophilen als auch hydrophoben Schadstoffen und bei starker Schmutzbelastung zeigen, ohne Anteile an den in Hautschutzmitteln üblicher Weise verwendeten anorganischen Pigmenten wie beispielsweise Oxide, z.B. Zink- und Titandioxid, Erdalkalicarbonate und -hydrogencarbonate sowie -sulfate, insbesondere deren Magnesium-, Calcium- und Strontiumsalze sowie Siliziumdioxid bzw. Kieselgelen, mineralische Silikate wie z.B. Aluminiumsilikate, Magnesium-Aluminium-Silicate, Bentonite, Kaoline und insbesondere Talkum zu enthalten, können die erfindungsgemäßen Hautschutzmittel bis zu 10 Gew.-%, bezogen auf das Gesamtgewicht des Hautschutzmittels, an anorganischen Pigmenten, insbesondere ausgewählt aus den zuvor genannten anorganischen Pigmenten als Zusatzstoffe enthalten, um beispielsweise das Hautgefühl zu verbessern oder einen Sonnenschutzfaktor einzuführen.

Durch den Zusatz von synthetischen und/oder natürlichen Gerbstoffen, vorzugsweise von 0,1 bis 5 Gew.-%, vorzugsweise 0,5 bis 2 Gew.-% und besonders bevorzugt 0,5 bis 1,5 Gew.-% bezogen auf die Gesamtzusammensetzung des Hautschutzmittels als weitere Komponente in den erfindungsgemäßen Hautschutzmitteln kann eine Verbesserung der Hautbarriere, insbesondere auch bei bereits vorgeschädigter Haut erreicht werden, wodurch eine wirksame Unterstützung der Hautregeneration bewirkt wird. Als synthetische oder natürliche Gerbstoffe können alle bekannten Gerbstoffe, die für kosmetische Anwendungen geeignet sind, in dem Hautschutzmittel enthalten sein. Vorzugsweise sind als synthetische Gerbstoffe solche ausgewählt aus der Gruppe umfassend Synthane, z.B.

Phenolsulfonsäure-Phenol-Harnstoff-Methanal-Kondensate, Natriumsalze von Phenolsulfosäure-Formaldehyd-Polykondensaten (TAMOL PP - Hersteller BASF AG, Ludwigshafen, oder die unter dem Handelsnamen Eucoriol^{®} - INCI-Bezeichnung: "Sodium Bischlorophenyl Sulfamine" (Hersteller: Evonik Stockhausen GmbH, Krefeld) enthalten. Natürliche Gerbstoffe sind z.B. Tannine oder sprühgetrocknete Hamamelisextrakte etc., wobei ein Zusatz in einer Menge von 0,1 bis 5 Gew.-%, bezogen auf die Gesamtmenge des Hautschutzmittels, von Hamamelis virginiana als natürlicher Gerbstoff besonders bevorzugt ist.

Die erfindungsgemäßen Hautschutzmittel sind vorzugsweise silikonfrei bzw. weisen vorteilhaft auch keine ökologisch bedenklichen Fluortenside als Inhaltsstoffe auf. Somit ist gewährleistet, dass bei der Weiterverarbeitung von Werkstücken keine Lackierungsfehler auftreten, wenn sich Hautschutzpräparatrückstände auf den zu lackierenden Werkstücken befinden würden. Somit sind die erfindungsgemäßen Mittel besonders vorteilhaft insbesondere in der Automobil- und Lackindustrie sowie der gummiverarbeitenden Industrie einsetzbar im Gegensatz zu Silikon- bzw. flourtensidhaltigen Hautschutzprodukten.

Erfindungsgemäß bevorzugt sind insbesondere Hautschutzmittel, die
a.) von 1 bis 12 Gew.-% mindestens eines Tensids,
b.) von 0,1 bis 5 Gew.-% mindestens eines Polyols und/oder Polyolderivats,
c.) 0 bis 5 Gew.-% kosmetische und/oder
   pharmazeutische Hilfs-, Zusatz- und/oder Wirkstoffe,
d.) 0,01 bis 5 Gew.-% mindestens einer barrierebildenden Komponente deren 1 gew.-%ige Lösung in Wasser einen nephelometrischen Trübungswert größer 40 (NTU) aufweist, und
e.) Wasser enthalten,
mit der Maßgabe, dass sich die Mengen der Komponenten a) bis e.) zu 100 Gew.-% ergänzen. Besonders bevorzugt sind diese Hautschutzmittel Silikon- und fluortensidfrei, ziehen leicht in die Haut ein und/oder, vorzugsweise und bilden keinen spürbaren Film auf der Haut.

Ganz besonders bevorzugte erfindungsgemäße Hautschutzmittel sind z. B. die in den unten beschriebenen Beispielen aufgeführten Hautschutzmittel, insbesondere jene, welche den Tabellen 1 bis 4 entnommen werden können.

Die erfindungsgemäßen Hautschutzmittel können als Schutzmittel gegen hydrophile (lipophobe) Schadstoffe und vorteilhafterweise sogar als Schutzmittel sowohl gegen hydrophobe (lipophile) als auch gegen hydrophile (lipophobe) Schadstoffe verwendet werden. Überdies kann die Verwendung des erfindungsgemäßen Hautschutzmittels die Hautreinigung erleichtern.

Die Erfindung betrifft überdies ein Verfahren zur Herstellung von Hautschutzmitteln, insbesondere gegen hydrophobe (lipophile) Schadstoffe, wobei eine barrierebildende Komponente zur Herstellung des Mittels dergestalt ausgewählt wird, dass der nephelometrische Trübungswert der barrierebildenden Komponente des Hautschutzmittels mittels Turbidimetrie bestimmt wird und mindestens eine barrierebildende Komponente, deren 1 gew.-%ige Lösung in Wasser einen nephelometrischen Trübungswert größer 40 (NTU) aufweist, zur Herstellung des Hautschutzmittels verwendet wird.

Erfindungsgemäß bevorzugt ist ein Verfahren, bei dem jeweils bezogen auf die Gesamtzusammensetzung des Hautschutzmittels, zur Herstellung des Hautschutzmittels
a.) von 1 bis 12 Gew.-% mindestens eines Tensids,
b.) von 0,1 bis 5 Gew.-% mindestens eines Polyols und/oder Polyolderivats,
c.) 0 bis 5 Gew.-% kosmetische und/oder
   pharmazeutische Hilfs-, Zusatz- und/oder Wirkstoffe,
d.) 0,01 bis 5 Gew.-% mindestens einer barrierebildenden Komponente deren 1 gew.-%ige Lösung in Wasser einen nephelometrischen Trübungswert größer 40 (NTU) aufweist, und
e.) Wasser,
wobei sich die Mengen der Komponenten a) bis e.) zu 100 Gew.-% ergänzen, eingesetzt werden.

Vorteilhaft ist, dass durch das erfindungsgemäße Verfahren Hautschutzmittel erhältlich sind, deren Eigenschafts- bzw. Schutzprofil bisher noch nicht erhalten werden konnte, nämlich die Gewährleistung einer Schutzwirkung sowohl vor öligen als auch wässrigen Noxen bei gleichzeitigem Schutz vor Schmutz, d.h. auch die Haftung stark haftender Verschmutzungen wie z.B. wasserunlöslicher Verschmutzungen durch Öle, Lacke, Graphit, Metallstäube, Klebestoffe, Kunstharze etc. kann durch die erfindungsgemäßen Hautschutzmittel vermindert werden. Besonders vorteilhaft ist hierdurch, dass eine anschließende Hautreinigung wesentlich schnellerund hautschonender durchgeführt werden kann.

Weiterhin vorteilhaft ist, dass für die erfindungsgemäßen Hautschutzmittel keine anorganischen Barrieremittel bzw. anorganische Pigmente wie z.B. Kaolin oder Talkum benötigt werden, die dem Anwender ein sehr unangenehmes trockenes Hautgefühl bei Hitze oder beim Tragen von Handschuhen vermitteln können, wodurch die Akzeptanz der Anwender hinsichtlich des Einsatzes von Hautschutzmitteln am Arbeitsplatz deutlich verbessert wird.

Die vorliegende Erfindung wird an Hand der Figuren 1 bis 7 näher erläutert, ohne darauf beschränkt zu sein.
Fig. 1 zeigt die Kalibrierkurve zur Bestimmung des nephelometrischen Trübungswertes (NTU-Wert). Darin bedeutet NG: Nachweisgrenze, EG: Erfassungsgrenze und BG: Bestimmungsgrenze.
Fig. 2 zeigt die Ergebnisse der Bestimmung der LDH-Aktivität. (3D-Hautmodell)
Fig. 3 zeigt die Ergebnisse der Zell-Viabilität (Standard- MTT-Test). (3D-Hautmodell)
Fig. 4 zeigt die Ergebnisse der Bestimmung der Interleukin 1α-Freisetzung. (3D-Hautmodell)
Fig. 5 zeigt die Ergebnisse der Bestimmung der repetitiven okklusiven Irritation.
Fig. 6 zeigt die Ergebnisse der Bestimmung der repetitiven okklusiven Irritation bei Wechselbelastung.
Fig. 7 zeigt die Ergebnisse der Handwaschtests.

Im Folgenden wird die Erfindung anhand von Beispielen, und Untersuchungen, insbesondere die Hautverträglichkeitsprüfung mit Hilfe des Duhring-Kammer-Testes, Prüfung auf Störung der Hautbarriere mit Hilfe des Tewameters und Prüfung der Reinigungskraft mit Hilfe des Handwaschtests und Ex vivo Verfahren wie z.B. das 3D-Hautmodell etc. sowie Figuren in Form von Diagrammen beschrieben.

Diese Erläuterungen sind lediglich beispielhaft und schränken die hier vorliegende Erfindung nicht ein. Die angegebenen Mengenangaben, Anteile und Prozentanteile sind, soweit nicht anders angegeben, auf das Gewicht und die Gesamtmenge bzw. auf das Gesamtgewicht der erfindungsgemäßen Hautschutzmittel bezogen.

### Beispiele:

### Prüfmethoden

### I. Bestimmung des nephelometrischen Trübungswertes (NTU-Wert)

### 1. Anwendungsbereich:

Messung der Trübung (nicht gelöster Bestandteile) in wässrigen Medien.

### 2. Kurzbeschreibung des Verfahrens:

Die Nephelometrie ist ein optisches Analysenverfahren zur Bestimmung des Feststoffanteiles in Suspensionen, Aerosolen bzw. trüben Dispersionen etc. Die Trübungsmessung hierbei beruht auf dem Faraday-Tyndall-Effekt, bei dem man die Intensität des Streulichtes misst.

Mit dem erfindungsgemäß verwendeten Turbidimeter wurde durch Messung der Intensitätsabnahme des Lichtes durch das streuende Medium die Trübung bestimmt (Turbidimetrie).

### 3. Geräte:

- HACH Turbidimeter 2100P ISO der Fa. Hach Company, Loveland, Colorado (USA)
- Wasseraufbereitungsgerät ELGA PURELAB Classic der Fa. ELGA Labwater, Celle (Deutschland).
- Rundküvetten mit Schraubverschluss Hach Cat. 24347-06
- beheizbarer Magnetrührer
- allgemeine Laborausrüstung

### 4. Chemikalien:

- Ultrareines Wasser
- StablCal Solution HACH < 0,1 NTU Cat. 26597-42
- StablCal Solution HACH 20 NTU Cat. 26601-42
- StablCal Solution HACH 100 NTU Cat. 26602-42
- StablCal Solution HACH 800 NTU Cat. 26605-42

### 5. Kalibrierung

Die Kalibrierung wurde unter Verwendung von Carrageenan (Viscarin^{®} PC209 von FMC Biopolymer, Philadelphia (USA)) durchgeführt.

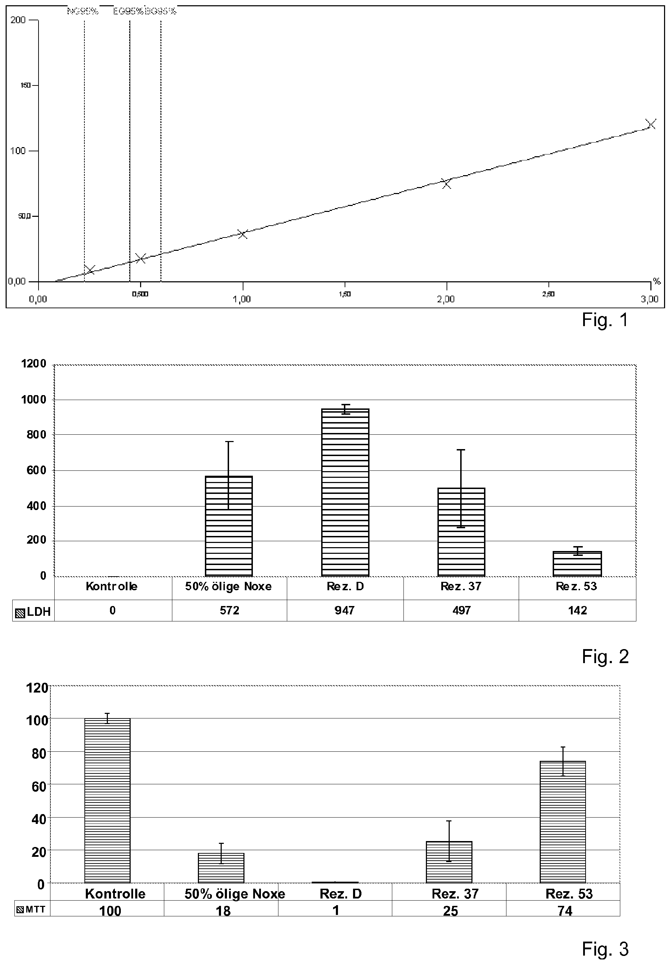

Die Kalibrierkurve ist in Fig. 1 dargestellt. Die Berechnung der Kalibrierung erfolgte nach DIN 38402 Teil 51:

| | |
|---|---|
| Ergebnisse für Funktion: Y= 40,25x - 2,92 | N= 5 Messwerte |
| Korrelationskoeffizient r | 0,999 |
| Reststandardabweichung sy | 2,427 |
| Verfahrensabweichung sx0 | 0,060 |
| Rel. Verfahrensstandardabweichung Vx0 | 4,467 |

### Kalibrierung des Messgerätes :

Die Kalibrierung des Messgerätes erfolgte nach Angaben und Anleitung des Geräteherstellers - Fa. Hach Company, Loveland, Colorado (USA) - mit Hilfe eines stabilisierten Formazinstandards, der von der Fa. Fach unter der Marke StablCal^{®} als standardisierte Lösung angeboten wird (siehe Punkt 4 "Chemikalien" und dort angegebene Katalognummern (cat.)). Hierbei war zu beachten ist, dass die Messlösungen luftblasenfrei waren.

### 6. Probenvorbereitung:

0,1, 1,0, 2,0 und/oder 3,0 g Probe (Tabellen 6a und 6b) wurden mit ultrareinem Wasser auf 100,0 g aufgefüllt und für 2 h auf dem Magnetrührer bei 500 rpm gerührt. Nach 3 Std. Standzeit wurde die Probe gemessen.

### 7. Durchführung der Bestimmung:

Die vorbereiteten Lösungen wurden luftblasenfrei in die dafür vorgesehenen Küvetten eingefüllt und mit dem zuvor frisch kalibrierten Turbidimeter gemessen. Der angezeigte Wert gab die Trübung in Form des nephelometrischen Trübungswertes (NTU-Wert) an.

### II. Ex vivo Verfahren: 3D - Hautmodell

3- dimensionale Hautmodelle besitzen einen analogen Aufbau wie die Humanhaut. Sie können auch als Testmodelle verwendet werden, um die Barrierereparatur nach Schädigung mit Modellirritantien zu untersuchen (vgl. A. zur Mühlen et al, "Using skin models to assess the effects of a protection cream on skin barrier function", Skin Pharmacol. Physiol 17 (4): 167-179).

Um die Beeinflussung von Wirkstoffen auf den Lipid-Gehalt der Haut zu analysieren, wurde unter Verwendung eines solchen 3-dimensionalen Hautmodells mit einem 1:1 Gemisch Undecylensäure: Ethylacetat als Modellirritans über die Analyse der Entzündungsparameter (IL-1α) und des LDH- und MTT-Wertes der Zustand der Hautmodelle charakterisiert. Nur wenn eine Schädigung im subtoxischen Bereich vorlag, d.h. durch die Modellnoxe keine massive Zellschädigung hervorgerufen wird, konnte eine Regeneration der Hautbarriere erfolgen.

Die Hautmodelle EST 1000 der Firma CellSystems^{®} Biotechnologie Vertrieb GmbH wurden in 6-well Zellkulturplatten mit je 900 µl "Maintenance Medium" überführt. Die Kulturen wurden bei 37 °C und 5 % CO₂ eine Stunde präinkubiert. Nach der Präinkubationsphase wurde die Aktivität der Lactatdehydrogenase (LDH) im Medium bestimmt und das komplette "Maintenance Medium" (900 µl) ausgetauscht. Anschließend wurde 30 µl 50% einer 1:1 Mischung Undecylensäure/Ethylacetat als Modellnoxe für ölige Noxen in PBS (Phosphatpuffer) auf die entsprechenden Hautmodelle pipettiert. Auf die Negativkontrollen
wurde nur PBS (PBS mit Ca und Mg, erhältlich von Biochrom, Berlin, Deutschland) gegeben. Nach 40 min wurde die LDH-Aktivität im Medium erneut bestimmt und alle Proben 3 x mit 500 µl PBS gewaschen. Danach fand erneut ein Medienwechsel statt. Anschließend wurden je 30 µl der entsprechenden Prüfsubstanz (siehe auch Plattenbelegung) auf die Oberfläche der Kulturen pipettiert.

Nach 24 h Inkubation wurde die LDH-Aktivität bestimmt. Die Hautmodelle wurden anschließend einem MTT-Test (24 und 48h) unterzogen.

Zum Test der Hautschutzprodukte wurde das Hautschutzmittel vor Applikation der Modellnoxe auf das Hautmodell mit einem sterilen Tupfer aufgetragen.

### Bestimmung der LDH- Freisetzung:

Der LDH-Test ist ein üblicher In-vitro-Zytotoxizitätstest, dessen Prinzip auf der Bestimmung der Enzymaktivität von Laktatdehydrogenase (LDH) im Zytoplasma basiert, das aus zerstörten oder beschädigten Zellen freigesetzt wird und somit im in vitro im Zellkulturmedium nachweisbar ist. Die Laktatdehydrogenase ist ein stabiles zytoplasmatisches Enzym, das sehr schnell in das Zellkulturmedium abgegeben wird, wenn die Zellmembran zerstört ist. Die LDH-Aktivität wird mittels eines enzymatischen Tests bestimmt. Hierbei gilt: Je höher der Grad der Zellschädigung ist, desto größer ist die Menge an freigesetztem LDH im Medium.

Die Quantifizierung der LDH-Aktivität wurde mit einem kommerziell erhältlichen LDH-Test-Kit der Fa. Roche Diagnostics, Mannheim, Deutschland durchgeführt und fand nach Herstellerangaben statt. Bei der Bestimmung der Aktivität der LDH wurde eine LDH- Verdünnungsreihe (LDH, Roche Diagnostics, Mannheim, Deutschland) als Standard mitgeführt.

### Bestimmung der Viabilität (Standard- MTT-Test):

Der MTT-Test ist ein Zytotoxizität-Test. Zellen werden in vitro mit dem namensgebenden Farbstoff, einem gelben Tetrazoliumsalz, behandelt, um ihre Lebensfähigkeit beziehungsweise den Anteil lebender Zellen im Vergleich zu einer Kontrollprobe von Zellen zu messen.

Die Vitalität der Zellen wurde mit dem Standard MTT-Test bestimmt. Hierbei wurde das gelbe Tertazolium-Salz MTT (Thiazolyl Blue Tetrazolium Bromide, Hersteller: Fa. Sigma)
durch mitochondriale Dehydrogenase metabolisch aktiver Zellen in einen blauen Formazan-Farbstoff reduziert, der anschließend photometrisch bestimmt wurde.

### Bestimmung des IL-1α-Gehaltes:

Interleukine (IL-x) sind zu den Zytokinen zählende Peptidhormone, d. h. sie sind körpereigene Botenstoffe der Zellen des Immunsystems, Interleukin-1 wird von Makrophagen, Endothelzellen, Fibroblasten und einigen anderen Zellen gebildet und ist ein entzündungsfördernder Signalstoff. IL1α spielt eine wichtige Rolle bei der Erhaltung der Hautbarrierfunktion. Die Quantifizierung der oben genannten Parameter wurde mit kommerziell erhältlichen Test-Kits (Interleukin 1α, R&D Systems GmbH, Wiesbaden- Norderstedt, Deutschland) durchgeführt und fand nach Herstellerangaben statt.

### III. Repetitiver Okklusiver Irritationstest: Hautschutzprodukte

Auf dem volaren Unterarm von Probanden wurden Testflächen/Felder markiert. Anschließend wurden die Ausgangswerte der Haut durch visuelle Begutachtung bestimmt. Nach dieser Bestimmung wurde dann das Hautschutzprodukt auf dem Unterarm aufgetragen. Ein Kontrollfeld und ein Feld als Positivstandard blieben unbehandelt bzw. frei.

Nach 10 min Einwirkzeit wurde überschüssiges Produkt abgetupft. Danach wurden Finn Kammern mit einer 1:1 Mischung Undecylensäure/Ethylacetat als Modellnoxe für ölige Noxen bzw. 2,5 %iger Natriumlaurylsulfatlösung (SDS) als Modellnoxe für wässrige Noxen auf die Testfelder gesetzt. Ein Testfeld blieb als Kontrollfeld frei.

Nach 30 min bei Tests auf Schutzwirkung des Hautschutzmittels gegen ölige Noxen bzw. 60 min bei Tests des Mittels gegen wässrige Noxen wurden die Finn Kammern abgenommen und mit Wasser abgespült. Am nächsten Tag vor dem nächsten Testzyklus wurden wieder der TEWL-Wert und der Corneometerwert bestimmt und visuell begutachtet. Die Testprozedur wurde 4 Tage lang wiederholt, am 5. Tag erfolgten dann die Messungen der Hautwerte (TEWL, Corneometer und visuelle Begutachtung).

Der Test auf häufig wechselnde Belastung der Haut wurde wie folgt durchgeführt:
Den Probanden wurde morgens 2,5 gew.-%ige Natriumlaurylsulfatlösung (SDS) auf die Haut aufgetragen, nachmittags erfolgte Auftragung eines Ethylacetat/Undecylensäure- Gemisches (1:1) für jeweils 30 min.

Die visuelle Bewertung erfolgte nach dem in der Tabelle 0 angegebenen Bewertungsschema.

**Tabelle 0: Bewertungsschema für die visuelle Bewertung.**

| Kriterien | Symptome | Punkte |
|---|---|---|
| R = Rötung | Negativ | 0 |
| (Erythem) | sehr leichtes, punktförmiges oder diffuses Erythem | 1 |
| | gut erkennbares, scharf begrenztes Erythem | 2 |
| | mittelstarkes Erythem | 3 |
| | starkes, feurig-rotes Erythem mit Ödem oder epidermalem Defekt (Bläschen, Nekrosen) | 4 |
| S = Schuppung | Negativ | 0 |
| | Trockenheit, Glanzeffekt | 1 |
| | feine Schuppung | 2 |
| | mäßige Schuppung | 3 |
| | starke Schuppung mit Abschilferungen | 4 |
| F = Fissuren | Negativ | 0 |
| (Risse) | sehr oberflächliche epidermale Separation | 1 |
| | einzelne oder mehrere weite Fissuren | 2 |
| | tiefe Fissuren mit Blutung und Exsudation | 3 |

Lag eine Bewertung zwischen zwei Bewertungspunkten, konnte zur weiteren Differenzierung die Beurteilung in 0,5er Schritten erfolgen. Anschließend wurde der Mittelwert aus der Summe der einzelnen Irritationswerte von R, S und F gebildet.

### V. Handwaschtest

Der orientierende Handwaschtest wurde mit 10 Probanden wie folgt durchgeführt:
- 1,2 g Testprodukt wurden aufgetragen und eingerieben
- 0,5 g Schmutz wurde auf den Handinnenflächen und auf den Handrücken verrieben
- 1 ml Wasser wurde zugefügt und 30 sec. gewaschen
- nochmals wurde 1 ml Wasser zugefügt und 30 sec. waschen gelassen
- unter fließendem kalten Wasser wurde abgespült
- die Beurteilung der Reinigungswirkung erfolgte nach der 6-Punkte-Skala

Als Modellschmutz wurde eine Mischung aus Öl, Fett und verschiedenen Pigmenten, die 54,15 Gew.-% Castrol-Motoröl, 18,05 Gew.-% Vaseline, 18,05 Gew.-% Adeps Lanae, 3,61 Gew.-% Graphit, 5,42 Gew.-% Flammruß und 0,72 Gew.-% Eisenoxid enthielt, verwendet.

Der Grad der Restverschmutzung auf der Handinnenfläche und dem Handrücken wurde im Anschluss an die Waschung nach der folgenden 6-Punkte-Skala bewertet.
0 =sauber
1 = leichte Restverschmutzung
2 = mittlere Restverschmutzung
3 = starke Restverschmutzung
4 = sehr starke Restverschmutzung
5 = kein Reinigungseffekt

Um eine bessere Differenzierung der Reinigungswirkung zu erhalten, erfolgte die Beurteilung durch den/die Durchführende(n) in 0,5er-Schritten.

### Beispielrezepturen:

Es wurden die in den nachfolgenden Tabellen 1 bis 5 aufgeführten Rezepturen hergestellt, wobei die Eignung der barrierebildenden Komponenten zuvor über die Bestimmung des nephelometrischen Trübungswertes (NTU-Wert) ermittelt worden war. Der NTU-Wert kann Tabelle 6 entnommen werden. Die Herstellung der erfindungsgemäßen Hautschutzmittel erfolgte dann durch Zusammenrühren der in den Tabellen genannten Komponenten mittels der in der Kosmetik bekannten üblichen Verfahren.

**Tabelle 1: Rezepturen**

| Inhaltsstoffe (INCI-Bezeichnungen) in Gew. -% | Rez. 1 | Rez. 2 | Rez. 3 | Rez. 4 | Rez. 5 |
|---|---|---|---|---|---|
| Ethylhexyl Stearate | 4,00 | 4,00 | 4,00 | 4,00 | 4,00 |
| Glyceryl Stearate SE | 3,50 | 3,50 | 3,50 | 3,50 | 3,50 |
| Glycerin | 3,00 | 3,00 | 3,00 | 3,00 | 3,00 |
| Cetearyl Alcohol | 2,50 | 2,50 | 2,50 | 2,50 | 2,50 |
| Sodium Bischlorophenyl Sulfamine | 2,10 | 2,10 | 2,10 | 2,10 | 2,10 |
| Isopropyl Palmitate | 2,00 | 2,00 | 2,00 | 2,00 | 2,00 |
| Glycol Distearate | 2,00 | 2,00 | 2,00 | 2,00 | 2,00 |
| Biosaccaride Gum-4 ¹⁾ | 5,00 | 2,00 | - | - | - |
| Chondrus Crispus (carrageenan)²⁾ | - | - | 2,00 | - | - |
| Polyurethane-32 ³⁾ | - | - | - | 1,00 | - |
| Sodium Alginate ⁴⁾ | - | - | - | - | 2,00 |
| Ceteareth-6 | 1,20 | 1,20 | 1,20 | 1,20 | 1,20 |
| Ceteareth-25 | 0,30 | 0,30 | 0,30 | 0,30 | 0,30 |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | ad 100 | ad 100 | ad 100 | ad 100 | ad 100 |
| 1) Glycofilm^{®} 1,5P von Solabia (ca. 1,5%ige Lösung) | | | | | |
| 2) Viscarin^{®} PC209 Carrageenan von FMC Biopolymer, Philadelphia (USA) bzw. FMC Europe, Brüssel (Belgien) | | | | | |
| 3) Baycusan^{®} C1003 von Bayer MaterialScience AG, Leverkusen (Deutschland) | | | | | |
| 4) Kelcosol^{®} von ISP | | | | | |

**Tabelle 1: Fortsetzung**

| Inhaltsstoffe (INCI-Bezeichnungen) in Gew. -% | Rez. 6 | Rez. 7 | Rez. 8 |
|---|---|---|---|
| Ethylhexyl Stearate | 4,00 | 4,00 | 4,00 |
| Glyceryl Stearate SE | 3,50 | 3,50 | 3,50 |
| Glycerin | 3,00 | 3,00 | 3,00 |
| Cetearyl Alcohol | 2,50 | 2,50 | 2,50 |
| Sodium Bischlorophenyl Sulfamine | 2,10 | 2,10 | 2,10 |
| Isopropyl Palmitate | 2,00 | 2,00 | 2,00 |
| Glycol Distearate | 2,00 | 2,00 | 2,00 |
| Hydrolyzed Sclerotium Gum ⁵⁾ | 2,00 | - | - |
| Polyglutamic acid ⁶⁾ | - | 2,00 | - |
| Polyvinyl alcohole ⁷⁾ | - | - | 2,00 |
| Ceteareth-6 | 1,20 | 1,20 | 1,20 |
| Ceteareth-25 | 0,30 | 0,30 | 0,30 |
| Phenoxyethanol | 0,72 | 0,72 | 0,72 |
| Methylparaben | 0,20 | 0,20 | 0,20 |
| Ethylparaben | 0,02 | 0,02 | 0,02 |
| Propylparaben | 0,02 | 0,02 | 0,02 |
| Butylparaben | 0,02 | 0,02 | 0,02 |
| Isobutylparaben | 0,02 | 0,02 | 0,02 |
| Parfüm | 0,20 | 0,20 | 0,20 |
| Aqua | ad 100 | ad 100 | ad 100 |
| 5) Tego Cosmo LSG von Evonik Goldschmidt, Essen | | | |
| 6) Tego Cosmo PGA von Evonik Goldschmidt, Essen | | | |
| 7) Mowiol 4-88 von Clariant | | | |
| 8) Viscarin 389 von FMC Biopolymer | | | |
| 9) Rewoteric AM C von Evonik Goldschmidt (40 -%ige Lösung) | | | |
| 10) Plantacare ACG 35 = Plantapon ACG LC von Cognis (35 -%ige Lösung) | | | |
| 11) Rewoteric AM KSF 40 von Evonik Goldschmidt (40 -%ige Lösung) | | | |
| 12) Rapithix A60 von ISP | | | |
| 13) Rewopol SB FA 30 B von Evonik Goldschmidt (33 -%ige Lösung) | | | |
| 14) Rapithix A100 von ISP | | | |
| 15) Baycusan C1000 von Bayer MaterialScience AG, Leverkusen (Deutschland) | | | |
| 16) Hostapon SCI von Clariant (85%ig) | | | |
| 17) Tegocel HPM 50 von Evonik Goldschmidt, Essen | | | |

**Tabelle 2: Rezepturen**

| Inhaltsstoffe (INCI-Bezeichnungen) in Gew. -% | Rez. 9 | Rez. 10 | Rez. 11 | Rez. 12 | Rez. 13 |
|---|---|---|---|---|---|
| Ethylhexyl Stearate | 4,00 | 4,00 | 4,00 | 4,00 | 4,00 |
| Glyceryl Stearate SE | 3,50 | 3,50 | 3,50 | 3,50 | 3,50 |
| Glycerin | 3,00 | 3,00 | 3,00 | 3,00 | 3,00 |
| Cetearyl Alcohol | 1,75 | 1,75 | 1,75 | 1,75 | 1,75 |
| Sodium Bischlorophenyl Sulfamine | 2,00 | 2,00 | 2,00 | 2,00 | 2,00 |
| Isopropyl Palmitate | 2,00 | 2,00 | 2,00 | 2,00 | 2,00 |
| Glycol Distearate | 2,00 | 2,00 | 2,00 | 2,00 | 2,00 |
| Biosaccaride Gum-4 ¹⁾ | 5,00 | 2,00 | - | - | - |
| Chondrus Crispus *²⁾* | - | - | 0,25 | - | - |
| Polyurethane-32 ³⁾ | - | - | - | 0,25 | - |
| Sodium Alginate ⁴⁾ | - | - | - | - | 0,25 |
| Ceteareth-6 | 1,20 | 1,20 | 1,20 | 1,20 | 1,20 |
| Ceteareth-25 | 0,30 | 0,30 | 0,30 | 0,30 | 0,30 |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | Ad 100 | ad 100 | ad 100 | ad 100 | ad 100 |

**Tabelle 3: Rezepturen**

| Inhaltsstoffe (INCI-Bezeichnungen) in Gew. -% | Rez. 14 | Rez. 15 | Rez. 16 | Rez. 17 | Rez. 18 |
|---|---|---|---|---|---|
| Ethylhexyl Stearate | 4,00 | 4,00 | 4,00 | 4,00 | 4,00 |
| Glyceryl Stearate SE | 3,50 | 3,50 | 3,50 | 3,50 | 3,50 |
| Glycerin | 3,00 | 3,00 | 3,00 | 3,00 | 3,00 |
| Cetearyl Alcohol | 0,500 | 1,75 | 1,75 | 1,75 | 1,75 |
| Sodium Bischlorophenyl Sulfamine | 2,00 | 2,00 | 2,00 | 2,00 | 2,00 |
| Isopropyl Palmitate | 2,00 | 2,00 | 2,00 | 2,00 | 2,00 |
| Sodium Stearoyl Glutamate | 1,44 | 1,44 | 1,44 | 1,44 | 1,44 |
| Cetearyl Alcohol | 0,50 | 0,50 | 0,50 | 0,50 | 0,50 |
| Biosaccaride Gum-4 *^{v}* | 5,00 | 1,00 | - | - | - |
| Chondrus Crispus *^{Z)}* | - | - | 0,25 | - | - |
| Polyurethane-32 ³⁾ | - | - | - | 0,25 | - |
| Sodium Alginate ⁴⁾ | - | - | - | - | 0,25 |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Sodium Chloride | 0,03 | 0,03 | 0,03 | 0,03 | 0,03 |
| Aqua | Ad 100 | ad 100 | ad 100 | ad 100 | ad 100 |

**Tabelle 3: Fortsetzung**

| Inhaltsstoffe (INCI-Bezeichnungen) in Gew. -% | Rez. 19 | Rez. 20 |
|---|---|---|
| Ceteareth-25 | 1,50 | 1,50 |
| Polyglyceryl-3 Methylglucose Distearate | 3,50 | 3,50 |
| Cetylstearylalkohol | 0,50 | 0,50 |
| Isopropylpalmitat | 2,00 | 2,00 |
| Isooctylstearat | 4,00 | 4,00 |
| Glycerin | 3,00 | 3,00 |
| Disodium Laureth Sulfosuccinate ¹³⁾ | | 8,00 |
| Natrium Benzoat | 0,60 | 0,60 |
| Milchsäure 80%ig | 0,30 | 0,30 |
| Zinksalicylate | 1,00 | 1,00 |
| Gingko | 0,10 | 0,10 |
| Xanthan Gum | 0,25 | 0,25 |
| Biosaccaride Gum-4 ¹⁾ | 2,00 | 2,00 |
| Aqua | ad 100 | ad 100 |

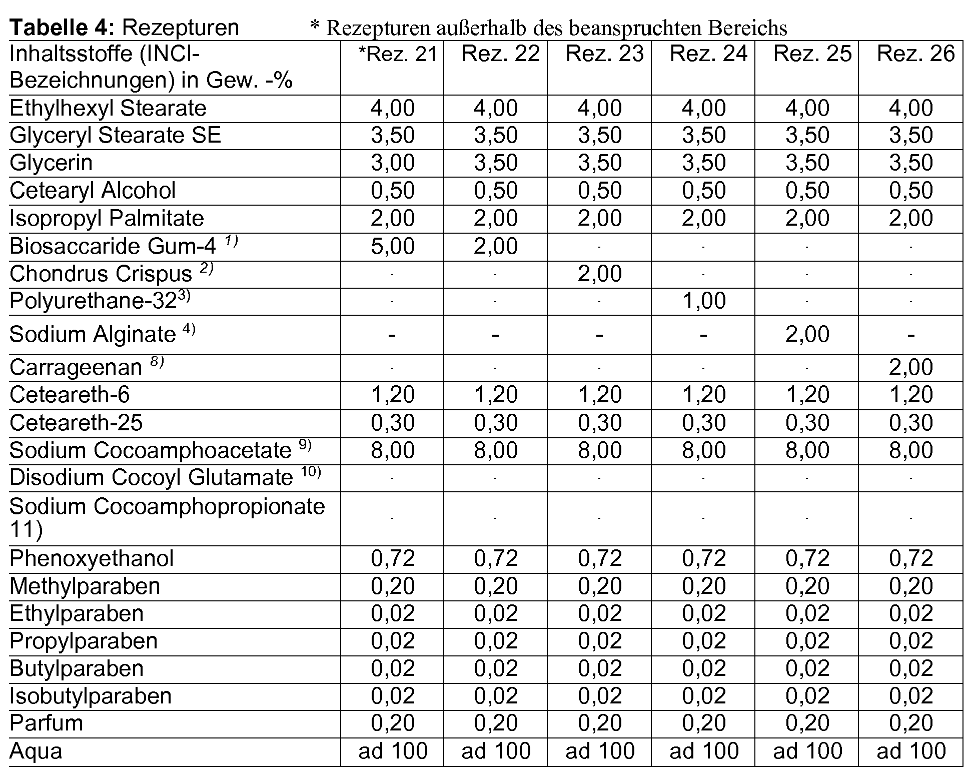

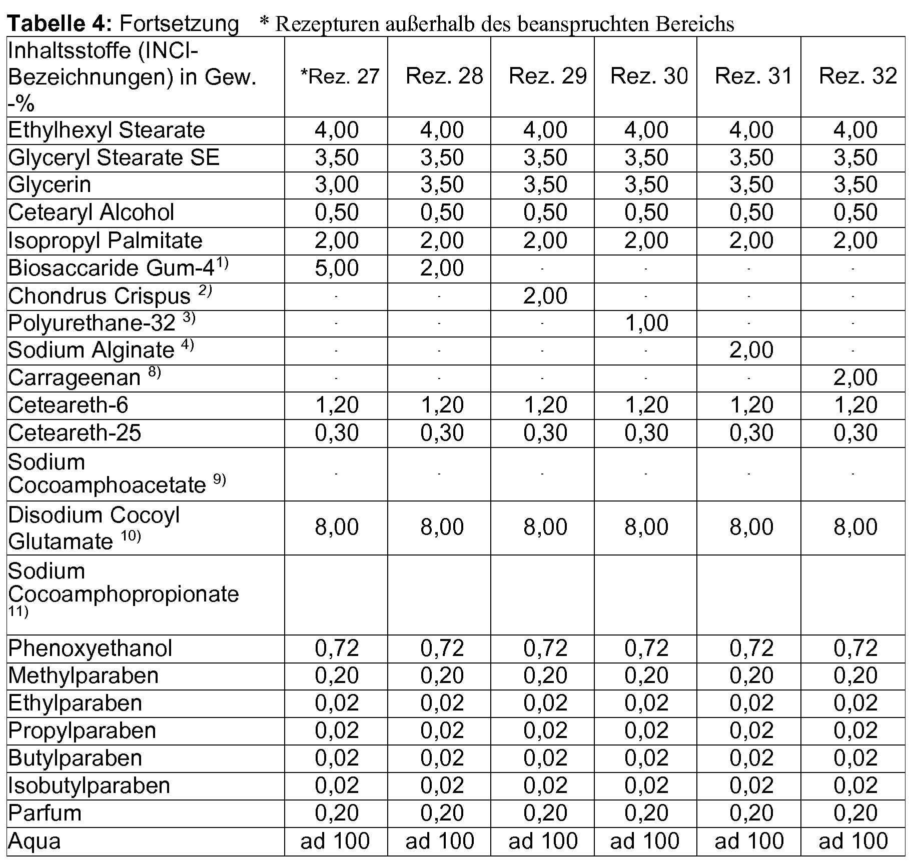

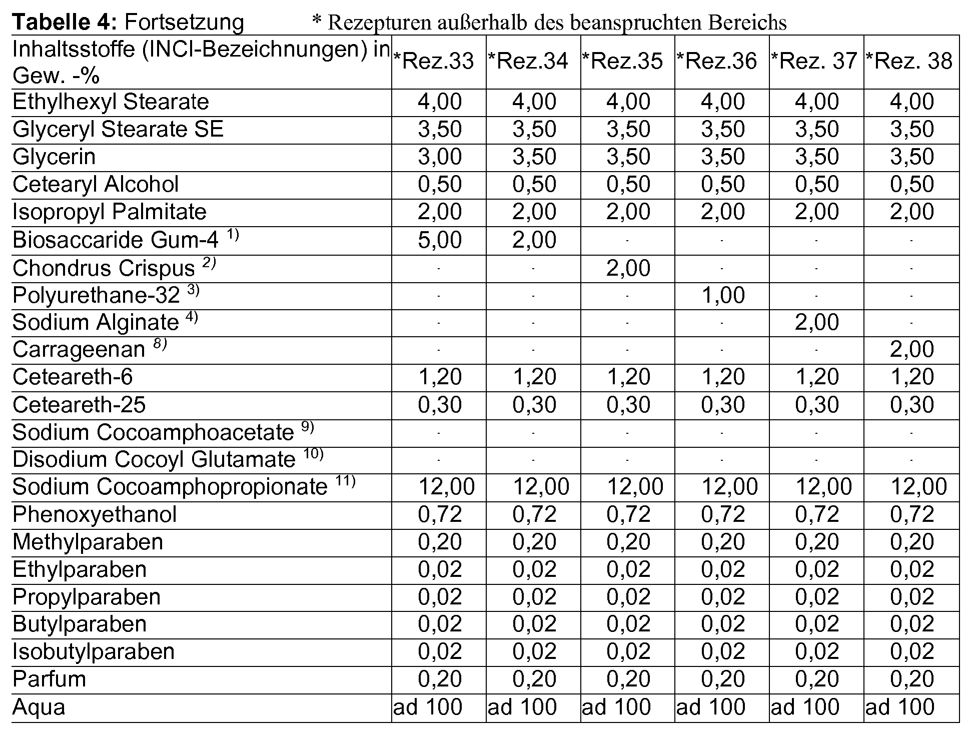

**Tabelle 4: Fortsetzung**

| Inhaltsstoffe (INCI-Bezeichnungen) in Gew. - % | Rez. 39 | Rez. 40 | Rez. 41 | Rez. 42 | Rez. 43 |
|---|---|---|---|---|---|
| Glycerin | 3,00 | 3,00 | 3,00 | 3,00 | 3,00 |
| Propylene Glycol | - | - | - | - | - |
| Capric/Caprylic Triglyceride | 2,00 | 2,00 | 2,00 | 2,00 | 2,00 |
| Sodium Polyacrylate | 0,87 | 0,87 | 0,87 | 0,87 | 0,87 |
| Hydrogenated Polydecene | 0,51 | 0,51 | 0,51 | 0,51 | 0,51 |
| Trideceth-6 | 0,09 | 0,09 | 0,09 | 0,09 | 0,09 |
| Biosaccaride Gum-4 ¹⁾ | 5,00 | 2,00 | - | - | - |
| Chondrus Crispus *²⁾* | - | - | 2,00 | - | - |
| Polyurethane-32 ³⁾ | - | - | - | 2,00 | - |
| Sodium Alginate ⁴⁾ | - | - | - | - | 2,00 |
| Sodium Cocoamphoacetate ⁹⁾ | 8,00 | 8,00 | 8,00 | 8,00 | 8,00 |
| Disodium Cocoyl Glutamate ¹⁰⁾ | - | - | - | - | - |
| Sodium Cocoamphopropionate ¹¹⁾ | - | - | - | - | - |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | ad 100 | ad 100 | ad 100 | ad 100 | ad 100 |

**Tabelle 4: Fortsetzung * Rezepturen außerhalb des beanspruchten Bereichs**

| Inhaltsstoffe (INCI-Bezeichnungen) in Gew. -% | *Rez. 44 | Rez. 45 | Rez. 46 | Rez. 47 | Rez. 48 |
|---|---|---|---|---|---|
| Glycerin | 3,00 | 3,00 | 3,00 | 3,00 | 3,00 |
| Propylene Glycol | - | - | - | - | - |
| Capric/Caprylic Triglyceride | 2,00 | 2,00 | 2,00 | 2,00 | 2,00 |
| Sodium Polyacrylate *¹²⁾⁾* | 0,87 | 0,87 | 0,87 | 0,87 | 0,87 |
| Hydrogenated Polydecene ¹²⁾ | 0,51 | 0,51 | 0,51 | 0,51 | 0,51 |
| Trideceth-6 *¹²⁾* | 0,09 | 0,09 | 0,09 | 0,09 | 0,09 |
| Biosaccaride Gum-4¹⁾ | 5,00 | 2,00 | - | - | 2,00 |
| Chondrus Crispus *²⁾* | - | - | 2,00 | - | - |
| Polyurethane-32 ³⁾ | - | - | - | 2,00 | - |
| Sodium Alginate ⁴⁾ | - | - | - | - | 2,00 |
| Sodium Cocoamphoacetate ⁹⁾ | - | - | - | - | - |
| Disodium Laureth Sulfosuccinate ¹³⁾ | 18,00 | 12,00 | 8,00 | 8,00 | 12,00 |
| Sodium Cocoamphopropionate ¹¹⁾ | - | - | - | - | - |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | ad 100 | ad 100 | ad 100 | ad 100 | ad 100 |

**Tabelle 4: Fortsetzung**

| Inhaltsstoffe (INCI-Bezeichnungen) in Gew. -% | Rez. 49 | Rez. 50 | Rez. 51 | Rez. 52 | Rez. 53 |
|---|---|---|---|---|---|
| Glycerin | 3,00 | 3,00 | 3,00 | 3,00 | 3,00 |
| Propylene Glycol | - | - | - | - | - |
| Capric/Caprylic Triglyceride | 2,00 | 2,00 | 2,00 | 2,00 | 2,00 |
| Sodium Polyacrylate ¹²⁾ | 0,87 | 0,87 | 0,87 | 0,87 | 0,87 |
| Hydrogenated Polydecene ¹²⁾ | 0,51 | 0,51 | 0,51 | 0,51 | 0,51 |
| Trideceth-6 ¹²⁾ | 0,09 | 0,09 | 0,09 | 0,09 | 0,09 |
| Biosaccaride Gum-4 ¹⁾ | 5,00 | 2,00 | - | - | - |
| Chondrus Crispus ²⁾ | - | - | 2,00 | - | - |
| Polyurethane-32 ³⁾ | - | - | - | 2,00 | - |
| Sodium Alginate ⁴⁾ | - | - | - | - | 2,00 |
| Sodium Cocoamphoacetate ⁹⁾ | - | - | - | - | - |
| Disodium Cocoyl Glutamate ¹⁰⁾ | - | - | - | - | - |
| Sodium Cocoamphopropionate ¹¹⁾ | 8,00 | 8,00 | 8,00 | 8,00 | 12,00 |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | ad 100 | ad 100 | ad 100 | ad 100 | ad 100 |

**Tabelle 4: Fortsetzung**

| Inhaltsstoffe (INCI- Bezeichnungen) in Gew. -% | Rez. 54 | Rez. 55 | Rez. 56 | Rez. 57 | Rez. 58 |
|---|---|---|---|---|---|
| Glycerin | | | | | |
| Propylene Glycol | 3,00 | 3,00 | 3,00 | 3,00 | 3,00 |
| Capric/Caprylic Triglyceride | 2,00 | 2,00 | 2,00 | 2,00 | 2,00 |
| Sodium Polyacrylate ¹²⁾ | 0,87 | 0,87 | 0,87 | 0,87 | 0,87 |
| Hydrogenated Polydecene *¹²⁾* | 0,51 | 0,51 | 0,51 | 0,51 | 0,51 |
| Trideceth-6 *¹²⁾* | 0,09 | 0,09 | 0,09 | 0,09 | 0,09 |
| Biosaccaride Gum-4 ¹⁾ | 5,00 | 2,00 | - | - | - |
| Chondrus Crispus *²⁾* | - | - | 2,00 | - | - |
| Polyurethane-32 ³⁾ | - | - | - | 2,00 | - |
| Sodium Alginate ⁴⁾ | - | - | - | - | 2,00 |
| Sodium Cocoamphoacetate⁹⁾ | - | - | - | - | - |
| Disodium Cocoyl Glutamate ¹⁰⁾ | - | - | - | - | - |
| Sodium Cocoamphopropionate | 8,00 | 8,00 | 8,00 | 8,00 | 8,00 |
| Phenoxyethanol | 0,72 | 0,72 | 0,72 | 0,72 | 0,72 |
| Methylparaben | 0,20 | 0,20 | 0,20 | 0,20 | 0,20 |
| Ethylparaben | 0,02 | 0,02 | 0,02 | 0,02 | 0,02 |
| Propylparaben | 0,02 | 0,02 | 0,02 | 0,02 | 0,02 |
| Butylparaben | 0,02 | 0,02 | 0,02 | 0,02 | 0,02 |
| Isobutylparaben | 0,02 | 0,02 | 0,02 | 0,02 | 0,02 |
| Parfum | 0,20 | 0,20 | 0,20 | 0,20 | 0,20 |
| Aqua | ad 100 | ad 100 | ad 100 | ad 100 | ad 100 |

**Tabelle 4: Fortsetzung * Rezepturen außerhalb des beanspruchten Bereichs**

| Inhaltsstoffe (INCI-Bezeichnungen) in Gew. - % | Rez. 59 | Rez. 60 | Rez. 61 | *Rez. 62 | Rez. 63 |
|---|---|---|---|---|---|
| Trisodium dicarboxymethyl alaninate | 0,01 | 0,01 | 0,01 | 0,01 | 0,01 |
| Phenoxyethanol | 0,94 | 0,94 | 0,94 | 0,94 | 0,94 |
| Propylenglykol | 3,50 | 3,50 | 3,50 | 3,50 | 3,50 |
| Capric/Caprylic Triglyceride | 2,00 | 2,00 | 2,00 | 2,00 | 2,00 |
| Parfum | 0,20 | 0,20 | 0,20 | 0,20 | 0,20 |
| Sodium polyacrylate ¹⁴⁾ | 1,00 | | 1,00 | 1,00 | 1,00 |
| Sodium Alginate ⁴⁾ | | 2,00 | | | |
| Biosaccaride Gum-4 ¹⁾ | 2,00 | 2,00 | 2,00 | 2,00 | 2,00 |
| Sodium Cocoamphoacetate ⁹⁾ | 12,00 | - | - | 18,00 | 6,00 |
| Disodium Laureth Sulfosuccinate ¹³⁾ | - | 12,00 | - | - | - |
| Sodium Cocoamphopropionate ¹¹⁾ | - | - | 12,00 | - | - |
| Aqua | ad 100 | ad 100 | ad 100 | ad 100 | ad 100 |

**Tabelle 4: Fortsetzung**

| Inhaltsstoffe (INCI- Bezeichnungen) in Gew. -% | Rez. 64 | Rez. 65 |
|---|---|---|
| Cremophor A6 (BASF) | 1,20 | 1,20 |
| Cremophor A25 (BASF) | 0,30 | 0,30 |
| Glyceryl Stearate SE | 3,50 | 3,50 |
| Glycerin | 3,00 | 3,00 |
| Cetylstearyl Alcohol | 2,50 | 2,50 |
| Ethylenglycoldistearate | 2,00 | 2,00 |
| Isopropyl Palmitate | 2,00 | 2,00 |
| Isooctylsterate | 4,00 | 4,00 |
| Polyurethane-32 ³⁾ | 2,00 | - |
| Polyurethane-34 ¹⁵⁾ | - | 2,00 |
| Phenoxyethanol | 0,72 | 0,72 |
| Methylparaben | 0,20 | 0,20 |
| Ethylparaben | 0,02 | 0,02 |
| Propylparaben | 0,02 | 0,02 |
| Butylparaben | 0,02 | 0,02 |
| Isobutylparaben | 0,02 | 0,02 |
| Parfum | 0,20 | 0,20 |
| Aqua | ad 100 | ad 100 |

**Tabelle 4: Fortsetzung * Rezepturen außerhalb des beanspruchten Bereichs**

| Inhaltsstoffe (INCI-Bezeichnungen) in Gew. -% | *Rez. 66 | *Rez. 67 |
|---|---|---|
| Decyl cocoate | 8,00 | 8,00 |
| Ethylhexyl palmitate | 5,00 | 5,00 |
| Cetyl ricinoleate | 2,00 | 2,00 |
| Cetearyl alcohol | 1,00 | 1,00 |
| Vitamin E acetate | 0,50 | 0,50 |
| Sucrose stearate | 2,00 | 2,00 |
| Cetearyl glucoside | 0,50 | 0,50 |
| Hydrolyzed sclerotium gum | 0,20 | 0,20 |
| Propylene glycol | 4,00 | 4,00 |
| Glycerin | 4,00 | 4,00 |
| Panthenol | 0,50 | 0,50 |
| Chondrus Crispus *²⁾* | 2,00 | 2,00 |
| Disodium Laureth Sulfosuccinate ¹³⁾ | 8,00 | |
| Sodium Cocoamphopropionate ¹¹⁾ | | 12,00 |
| Phenoxyethanol | 0,72 | 0,72 |
| Methylparaben | 0,20 | 0,20 |
| Ethylparaben | 0,02 | 0,02 |
| Propylparaben | 0,02 | 0,02 |
| Butylparaben | 0,02 | 0,02 |
| Isobutylparaben | 0,02 | 0,02 |
| Parfum | 0,20 | 0,20 |
| Aqua | ad 100 | ad 100 |

**Tabelle 5: Nicht erfindungsgemäße Rezepturen**

| Inhaltsstoffe (INCI-Bezeichnungen) in Gew. -% | Rez. A | Rez. B | Rez. C | Rez. D |
|---|---|---|---|---|
| Tale | 21,00 | 21,00 | 21,00 | 21,00 |
| Glycerin | 7,00 | 7,00 | 7,00 | 7,00 |
| Silica | 3,00 | 3,00 | 3,00 | 3,00 |
| Glyceryl Stearate | 2,90 | 2,90 | 2,90 | 2,90 |
| Laureth-10 | 2,00 | 2,00 | 2,00 | 2,00 |
| Bentonite | 0,99 | 0,99 | 0,99 | 0,99 |
| Sodium Phosphate | 0,46 | 0,46 | 0,46 | 0,46 |
| Lactic Acid | 0,20 | 0,20 | 0,20 | 0,20 |
| Potassium Sorbate | 0,30 | 0,30 | 0,30 | 0,30 |
| Dioctyl Sodium Sulfosuccinate | 0,02 | 0,02 | 0,02 | 0,02 |
| Silver Chloride | 0,002 | 0,002 | 0,002 | 0,002 |
| Propylene Glycol | 0,002 | 0,002 | 0,002 | 0,002 |
| Titanium Dioxide | 0,003 | 0,003 | 0,003 | 0,003 |
| Sodium Cocoamphoacetate ⁹⁾ | 8,00 | - | - | - |
| Disodium Cocoyl Glutamate ¹⁰⁾ | - | 8,00 | - | - |
| Sodium Cocoamphopropionate¹¹⁾ | - | - | 8,00 | - |
| Sodium cocoyl isethionat¹⁶⁾ | - | - | - | 9,00 |
| Parfum | 0,20 | 0,20 | 0,20 | 0,20 |
| Aqua | ad 100 | ad 100 | ad 100 | ad 100 |

**Tabelle 5: Fortsetzung**

| Inhaltsstoffe (INCI-Bezeichnungen) in Gew. -% | Rez. E |
|---|---|
| Ethylhexyl Stearate | 4,00 |
| Glyceryl Stearate SE | 3,50 |
| Glycerin | 3,00 |
| Cetearyl Alcohol | 2,50 |
| Sodium Bischlorophenyl Sulfamine | 2,10 |
| Isopropyl Palmitate | 2,00 |
| Glycol Distearate | 2,00 |
| Hydroxypropylcellulose ¹⁷⁾ | 2,00 |
| Ceteareth-6 | 1,20 |
| Ceteareth-25 | 0,30 |
| Phenoxyethanol | 0,72 |
| Methylparaben | 0,20 |
| Ethylparaben | 0,02 |
| Propylparaben | 0,02 |
| Butylparaben | 0,02 |
| Isobutylparaben | 0,02 |
| Parfum | 0,20 |
| Aqua | ad 100 |

**Tabelle 6: Nephlometrischer Trübungswert (NTU, Nephelometrie Turbidity Unit)**

| **Probe (INCI-Bezeichnungen)** | **1 gew.-%ige Lösung** | **2 gew-%ige Lösung** | **3 gew-%ige Lösung** |
|---|---|---|---|
| Sodium Alginate ⁴⁾ **(Kelcosol^{®} von ISP)** | **90** | **160** | **192** |
| Sodium Alginate **(Algogel^{®} 3020 von Degussa Texturant Systems France SAS)** | **72** | **152** | **202** |
| Biosaccaride Gum-4 *^{v}* **(Glycofilm^{®} 1,5 P (H₂0 98%) von C.H. Erbslöh KG, Krefeld)** | **66** | **112** | **---*** |
| Chondrus Crispus (carrageenan)^{A} **(Viscarin^{®} PC209 Carrageenan von FMC Biopolymer)** | **48** | **84** | **120** |
| Xanthan Gum **(Rhodicare S)** | **37** | **78** | **---*** |
| Hydrolyzed Sclerotium Gum *^{b)}* **Tego Cosmo LSG** | **21** | **41** | **92** |
| Sodium Carboxymethylcellulose **WALOCEL^{®} CRT 2000** | **15** | **29** | **---*** |
| Hydroxypropycellulose **Klucel^{®}** | **10** | **21,0** | **---*** |
| Hydroxypropyl Methylcellulose¹⁷⁾ **Tegocel HPM 50** | **5** | **9** | **14** |
| Polyglutamic acid ⁶⁾ **Tego Cosmo PGA** | **4,9** | **9,5** | **14,4** |
| Polyvinyl alcohole ⁷⁾ **Mowiol^{®} 4-88 (Fa. Clariant)** | **0,7** | **1,0** | **1,5** |

**Tabelle 6: Fortsetzung**

| **Probe** | **0,1 gew-%ige. Lösung/ WS** | **1 gew-%ige Lösung/ WS** |
|---|---|---|
| Polyurethane-34 ^{1b)} **Baycusan^{®} C 1000 (H₂0: 56 %)** | **173** | **> 1000 **** |
| Polyurethane-32 **Baycusan^{®} C 1003 (H₂0: 48,4%)** | **510** | **> 1000 **** |
| Sodium polyacrylate ¹⁴⁾ **Rapithix^{®} A 100 (H₂0: 0,4%)**** | **443** | **_*** |
| * Messung nicht möglich (Lufteinschlüsse, zu hohe Viscosität, Wassergehalt). | | |
| ** Ein Messwert > 1000 ist außerhalb des vorgegebenen Messbereichs des Gerätes. | | |

### Prüfergebnisse:

### 1. Bestimmung der LDH-Aktivität

Erfindungsgemäße und nicht erfindungsgemäß Rezepturen wurden aufgetragen und anschließend die LDH-Aktivität gemessen. In einer Blindprobe wurden die Noxen ohne vorheriges Aufbringen einer Rezeptur aufgebracht und die LDH-Aktivität bestimmt. Die Ergebnisse der Messungen können Fig. 2 sowie der nachfolgenden Tabelle 7 entnommen werden.

**Tabelle 7: Ergebnis der Bestimmung der LDH-Aktivität**

| | LDH | Standardabweichung |
|---|---|---|
| Kontrolle | 0 | 0 |
| 50 % ölige Noxen | 572 | 194 |
| Rez. D | 947 | 28 |
| Rez. 37 | 497 | 221 |
| Rez. 53 | 142 | 22 |

Die erfindungsgemäßen Rezepturen 37 und 53 zeigten eine deutlich verringerte Menge an freigesetztem LDH, d.h. diese Rezepturen zeigten eine signifikante Schutzwirkung im Gegensatz zur nicht erfindungsgemäßen Rezeptur D.

### 2. Bestimmung der Zell-Viabilität (Standard- MTT-Test)

Für erfindungsgemäße und nicht erfindungsgemäße Rezepturen sowie eine Blindprobe wurde die Zell-Viabilität gemessen. Die Ergebnisse der Messungen können Fig. 3 sowie der nachfolgenden Tabelle 8 entnommen werden.

**Tabelle 8: Ergebnis der Bestimmung der Zell-Viabilität**

| | MTT | Standardabweichung |
|---|---|---|
| Kontrolle | 100 | 3,1 |
| 50 % ölige Noxen | 18 | 6,1 |
| Rez. D | 1 | 0,1 |
| Rez. 37 | 25 | 12,3 |
| Rez. 53 | 74 | 8,8 |

Die erfindungsgemäßen Rezepturen 37 und 53 zeigten eine deutlich erhöhte Zellviabilität, d.h. diese Rezepturen zeigten eine signifikante Schutzwirkung im Gegensatz zur nicht erfindungsgemäßen Rezeptur D.

### 3. Bestimmung der Interleukin 1α-Freisetzung

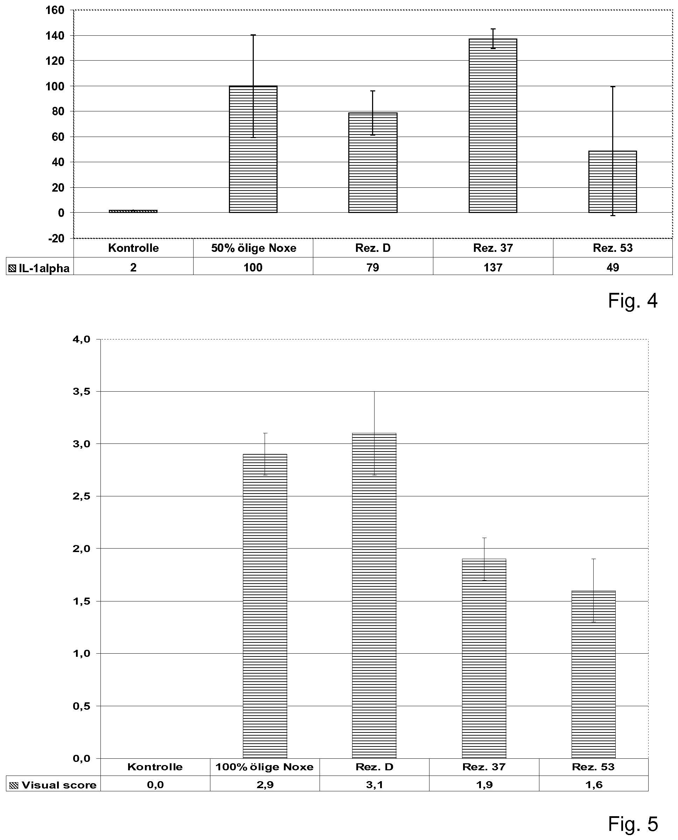

Für erfindungsgemäße und nicht erfindungsgemäße Rezepturen sowie eine Blindprobe wurde wie oben beschrieben die Interleukin 1α-Freisetzung bestimmt. Die Ergebnisse der Messungen können Fig. 4 sowie der nachfolgenden Tabelle 9 entnommen werden.

**Tabelle 9: Ergebnis der Bestimmung der Interleukin 1α-Freisetzung**

| | Interleukin 1α | Standardabweichung |
|---|---|---|
| Kontrolle | 2 | 0,2 |
| 50 % ölige Noxen | 100 | 40,5 |
| Rez. D | 79 | 17,4 |
| Rez. 37 | 137 | 7,8 |
| Rez. 53 | 49 | 50,9 |

Die nicht erfindungsgemäßen Rezeptur D zeigte keine Entzündungsreaktion, da die Zellen bereits alle abgestorben waren; eine Regeneration der Hautbarriere war nicht mehr möglich. Die erfindungsgemäße Rezeptur 37 zeigte eine leichte Erzündungsreaktion analog zu einer leichten Schädigung. Rezeptur 53 zeigte entsprechend der kaum vorhandenen Schädigung, so gut wie keine Entzündungsreaktion.

### 4. Bestimmung der repetitiven okklusiven Irritation

Für erfindungsgemäße und nicht erfindungsgemäße Rezepturen sowie eine Blindprobe wurde wie oben beschrieben die repetitive okklusive Irritation bestimmt. Die Ergebnisse der Messungen können Fig. 5 sowie der nachfolgenden Tabelle 10 entnommen werden.

**Tabelle 10: Ergebnis der Bestimmung der repetitiven okklusiven Irritation**

| | Visual Score | Standardabweichung |
|---|---|---|
| Kontrolle | 0,0 | 0,0 |
| 50 % ölige Noxen | 2,9 | 0,2 |
| Rez. D | 3,1 | 0,4 |
| Rez. 37 | 1,9 | 0,2 |
| Rez. 53 | 1,6 | 0,3 |

Wie Tabelle 10 entnommen werden kann, wurden mit den erfindungsgemäßen Rezepturen deutlich geringere Visual Score Werte erhalten als mit der nicht erfindungsgemäßen Rezeptur D.

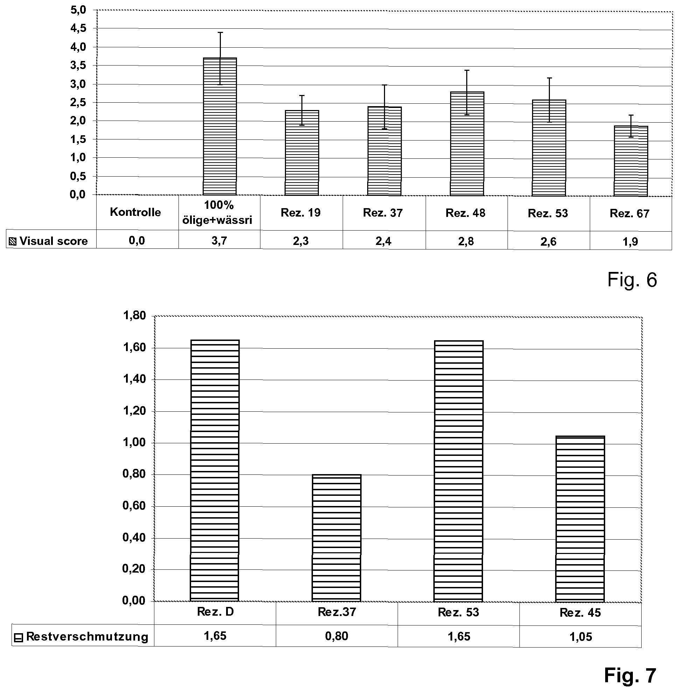

Für einige erfindungsgemäße Rezepturen sowie eine Blindprobe wurde wie oben beschrieben die repetitive okklusive Irritation bei Wechselbelastung bestimmt. Die Ergebnisse der Messungen können Fig. 6 sowie der nachfolgenden Tabelle 11 entnommen werden.

**Tabelle 11: Ergebnis der Bestimmung der repetitiven okklusiven Irritation bei Wechselbelastung**

| | Visual Score | Standardabweichung |
|---|---|---|
| Kontrolle | 0,0 | 0,0 |
| 100 % ölige Noxen + wässrige Noxen | 3,7 | 0,7 |
| Rez. 19 | 2,3 | 0,4 |
| Rez. 37 | 2,4 | 0,6 |
| Rez. 48 | 2,8 | 0,6 |
| Rez. 53 | 2,6 | 0,6 |
| Rez. 67 | 1,9 | 0,3 |

Der Tabelle 11 kann entnommen werden, dass durch die Verwendung der erfindungsgemäßen Hautschutzmittel bei einer abwechselnden Einwirkung von öligen und wässrigen Noxen nur eine geringe Schädigung zu beobachten ist.

### 5. Handwaschtests

Für erfindungsgemäße und nicht erfindungsgemäße Rezepturen sowie eine Blindprobe wurden Handwaschtest wie oben beschrieben durchgeführt. Die Ergebnisse der Test können Fig. 7 sowie der nachfolgenden Tabelle 12 entnommen werden.

**Tabelle 12: Ergebnisse der Handwaschtests**

| | Restverschmutzung |
|---|---|
| Rez. D | 1,65 |
| Rez. 37 | 0,8 |
| Rez. 45 | 1,65 |
| Rez. 53 | 1,05 |

Die Handwaschtests zeigen, dass die erfindungsgemäßen Hautschutzmittel auch die Reinigung der Haut erleichtern bzw. fördern.

## Patentansprüche

1. Hautschutzmittel, **dadurch gekennzeichnet, dass** das Mittel jeweils bezogen auf die Gesamtzusammensetzung des Hautschutzmittels,
a) von 1 bis 12 Gew.-% mindestens eines Tensids,
b) von 0,1 bis 5 Gew.-% mindestens eines Polyols und/oder Polyolderivats,
c) von 0 bis 5 Gew.-% kosmetische und/oder pharmazeutische Hilfs-, Zusatz- und/oder Wirkstoffe,
d) von 0,01 bis 5 Gew.-% einer barrierebildenden Komponente, die aus Gummi arabicum, Johannisbrotkernmehl, Traganth, Karayagummi, Guarkernmehl, Pektin, Gellangummi, Carrageen, Agar, Alginaten, Chondrus crispus (Carrageen) und Biosaccharide Gum-4 oder einem Gemisch aus zwei oder
mehreren dieser Polymere ausgewählt wird und
e) Wasser,
enthält, wobei sich die Mengen der Komponenten a.) bis e.) zu 100 Gew.-% ergänzen, und wobei eine 1 gew.-%ige Lösung der barrierebildenden Komponente in Wasser einen nephelometrischen Trübungswert, bestimmt mittels Turbidimetrie, größer 40 (NTU) aufweist.

2. Hautschutzmittel nach Anspruch 1, **dadurch gekennzeichnet, dass** der Anteil der mindestens einen barrierebil- denden Komponente 0,03 bis 5 Gew.-% und besonders bevorzugt 0,03 bis 3 Gew.-% bezogen auf die Gesamtzu- sammensetzung des Hautschutzmittels beträgt.

3. Hautschutzmittel nach Anspruch 1 oder 2, **dadurch gekennzeichnet, dass** das mindestens eine Tensid aus der Gruppe umfassend Aminosäuretenside, Acylpeptide, Sarcosinate, Acyltauride, Isethionate, Sulfosuccinate, zwitter- ionische und amphotere Tenside oder aus der Gruppe der Polyethylen- und/oder Polypropylenglykolderivate aus- gewählt wird.

4. Hautschutzmittel nach einem der Ansprüche 1 bis 3, **dadurch gekennzeichnet, dass** das mindestens eine Polyol und/oder Polyolderivat Glyzerin und/oder 1,2-Propandiol ist.

5. Hautschutzmittel nach einem der Ansprüche 1 bis **4, dadurch gekennzeichnet, dass** das Mittel 0,05 bis 5 Gew.-
%, bezogen auf die Gesamtzusammensetzung, eines oder mehrerer Hilfs-, Zusatz- und/oder Wirkstoffe enthält.

6. Hautschutzmittel nach einem der Ansprüche 1 bis 5, **dadurch gekennzeichnet, dass** das Mittel fluortensid- und silikonfrei ist.

7. Hautschutzmittel nach einem der Ansprüche 1 bis 6 zur Verwendung als Schutzmittel gegen hydrophile (lipophobe)
Schadstoffe.

8. Hautschutzmittel nach einem der Ansprüche 1 bis 6 zur Verwendung als Schutzmittel gegen hydrophobe (lipophile) Schadstoffe sowie hydrophile (lipophobe) Stoffe.

9. Hautschutzmittel nach einem der Ansprüche 1 bis 6 zur Verwendung für die Erleichterung der nicht therapeutischen Hautreinigung nach Verschmutzung.

10. Verfahren zur Herstellung von Hautschutzmitteln, insbesondere gegen hydrophobe (lipophile) Schadstoffe, wobei eine Zusammensetzung nach einem der Ansprüche 1 bis 6 zur Herstellung des Hautschutzmittels eingesetzt wird

## Claims

1. Skin protection composition, **characterized in that** the composition contains, based in each case on the overall skin protection composition,
a) from 1% to 12% by weight of at least one surfactant,
b) from 0.1% to 5% by weight of at least one polyol and/or polyol derivative,
c) from 0% to 5% by weight of cosmetic and/or pharmaceutical auxiliaries, additives and/or actives,
d) from 0.01% to 5% by weight of a barrier-forming component selected from gum arabic, locust bean gum, tragacanth, karaya gum, guar seed flour, pectin, gellan gum, carrageenan, agar, alginates, Chondrus crispus (carrageenan) and Biosaccharide Gum-4 or a mixture of two or
more of these polymers and
e) water,
where the amounts of components a.) to e.) add up to 100% by weight, and where a 1% by weight solution of the barrier-forming component in water has a nephelometric turbidity value, determined by turbidimetry, of greater than 40 (NTU).

2. Skin protection composition according to Claim 1, **characterized in that** the proportion of the at least one barrier-forming component is 0.03% to 5% by weight and more preferably 0.03% to 3% by weight, based on the overall skin protection composition.

3. Skin protection composition according to Claim 1 or 2, **characterized in that** the at least one surfactant is selected from the group comprising amino acid surfactants, acyl peptides, sarcosinates, acyl taurides, isethionates, sulfosuccinates, zwitterionic and amphoteric surfactants, or the group of polyethylene glycol derivatives and/or polypropylene glycol derivatives.

4. Skin protection composition according to any of Claims 1 to 3, **characterized in that** the at least one polyol and/or polyol derivative is glycerol and/or propane-1,2-diol.

5. Skin protection composition according to any of Claims 1 to 4, **characterized in that** the composition contains 0.05% to 5% by weight, based on the overall composition, of one or more auxiliaries, additives and/or actives.

6. Skin protection composition according to any of Claims 1 to 5, **characterized in that** the composition is free of fluorosurfactants and silicones.

7. Skin protection composition according to any of Claims 1 to 6 for use as protection composition against hydrophilic (lipophobic) harmful substances.

8. Skin protection composition according to any of Claims 1 to 6 for use as protection composition against hydrophobic (lipophilic) harmful substances and hydrophilic (lipophobic) substances.

9. Skin protection composition according to any of Claims 1 to 6 for use for facilitation of non-therapeutic skin cleansing after soiling.

10. Method of producing skin protection compositions, especially to counter hydrophobic (lipophilic) harmful substances, using a composition according to any of Claims 1 to 6 for production of the skin protection composition.

## Revendications

1. Agent de protection de la peau, **caractérisé en ce que** l'agent contient, à chaque fois par rapport à la composition totale de l'agent de protection de la peau,
a) de 1 à 12 % en poids d'au moins un tensioactif,
b) de 0,1 à 5 % en poids d'au moins un polyol et/ou dérivé de polyol ;
c) de 0 à 5 % en poids d'adjuvants, additifs et/ou substances actives cosmétiques et/ou pharmaceutiques,
d) de 0,01 à 5 % en poids d'un composant formant barrière qui est choisi parmi la gomme arabique, la farine de graines de caroube, la gomme adragante, la gomme karaya, la gomme de guar, la pectine, la gomme gellane, le carraghénane, l'agar-agar, les alginates, Chondrus crispus (carraghénane) et le Biosaccharide-Gum-4 ou un mélange de deux ou
plus de deux de ces polymères et
e) de l'eau,
dans lequel les quantités des composants a.) à e.) totalisent 100 % en poids, et dans lequel une solution à 1 % en poids du composant formant barrière dans de l'eau a une valeur de turbidité néphélométrique, déterminée par turbidimétrie, supérieure à 40 (NTU).

2. Agent de protection de la peau selon la revendication 1, **caractérisé en ce que** la proportion de l'au moins un composant formant barrière est de 0,03 à 5 % en poids, et de manière particulièrement préférée de 0,03 à 3 % en poids, par rapport à la composition totale de l'agent de protection de la peau.

3. Agent de protection de la peau selon la revendication 1 ou 2, **caractérisé en ce que** ledit au moins un tensioactif est choisi dans le groupe comprenant des tensioactifs de type acide aminé, des acylpeptides, des sarcosinates, des acyltaurides, des iséthionates, des sulfosuccinates, des tensioactifs zwitterioniques et amphotères ou dans le groupe des dérivés de polyéthylèneglycol et/ou polypropylèneglycol.

4. Agent de protection de la peau selon l'une quelconque des revendications 1 à **3, caractérisé en ce que** l'au moins un polyol et/ou dérivé de polyol est la glycérine et/ou le 1,2-propanediol.

5. Agent de protection de la peau selon l'une quelconque des revendications 1 à **4, caractérisé en ce que** l'agent contient 0,05 à 5 % en poids, par rapport à la composition totale, d'un(e) ou plusieurs adjuvants, additifs et/ou substances actives.

6. Agent de protection de la peau selon l'une quelconque des revendications 1 à **5, caractérisé en ce que** l'agent est exempt de tensioactif fluoré et de silicone.

7. Agent de protection de la peau selon l'une quelconque des revendications 1 à 6, destiné à être utilisé comme agent de protection contre les substances nocives hydrophiles (lipophobes).

8. Agent de protection de la peau selon l'une quelconque des revendications 1 à 6, destiné à être utilisé comme agent de protection contre les substances nocives hydrophobes (lipophiles) ainsi que contre les substances hydrophiles (lipophobes).

9. Agent de protection de la peau selon l'une quelconque des revendications 1 à 6, destiné à être utilisé pour faciliter le nettoyage non thérapeutique de la peau après salissure.

10. Procédé de préparation d'agents de protection de la peau, en particulier contre des substances nocives hydrophobes (lipophiles), **caractérisé en ce que** l'on utilise pour la préparation de l'agent de protection de la peau une composition selon l'une quelconque des revendications 1 à 6.
